(19) **Europäisches Patentamt**

**European Patent Office**

**Office européen des brevets**

(11) **EP 1 270 015 A2**

(12) **EUROPEAN PATENT APPLICATION**

(43) Date of publication:
**02.01.2003   Bulletin 2003/01**

(51) Int Cl.7: **A61K 47/48**

(21) Application number: **02254497.7**

(22) Date of filing: **26.06.2002**

(84) Designated Contracting States:
**AT BE CH CY DE DK ES FI FR GB GR IE IT LI LU
MC NL PT SE TR**
Designated Extension States:
**AL LT LV MK RO SI**

(30) Priority:  **29.06.2001  JP  2001198985**

(71) Applicant: **Sankyo Company Limited
Chuo-ku, Tokyo 103-8426 (JP)**

(72) Inventors:
  • **Yamamoto, Shinichi, Sankyo Company Limited
Shinagawa-ku, Tokyo 140-8710 (JP)**
  • **Okada, Junichi, Sankyo Company Limited
Shinagawa-ku, Tokyo 140-8710 (JP)**
  • **Kurihara, Atsushi, Sankyo Company Limited
Shinagawa-ku, Tokyo 140-8710 (JP)**

  • **Numazawa, Taku, Sankyo Company Limited
Shinagawa-ku, Tokyo 140-8710 (JP)**
  • **Kondo, Junichi, Sankyo Company Limited
Shinagawa-ku, Tokyo 140-8710 (JP)**
  • **Tsuda, Eisuke, Sankyo Company Limited
Shinagawa-ku, Tokyo 140-8710 (JP)**
  • **Mochizuki, Shinichi, Sankyo Company Limited
Shinagawa-ku, Tokyo 140-8710 (JP)**
  • **Nishi, Hirotaka, Sankyo Company Limited
Shinagawa-ku, Tokyo 140-8710 (JP)**
  • **Miyazaki, Hideki, Sankyo Company Limited
Shinagawa-ku, Tokyo 140-8710 (JP)**

(74) Representative: **Gibson, Christian John Robert
MARKS & CLERK,
57/60 Lincoln's Inn Fields
London WC2A 3LS (GB)**

(54) **A complex comprising OCIF and Polysaccharide**

(57)    A novel complex comprising at least one substance selected from the group consisting of osteoclastogenesis inhibitory factor (OCIF), analogues thereof and variants thereof, which is bound to at least one substance selected from the group consisting of polysaccharides and derivatives thereof shows prolonged retention in the bloodstream after administration making it useful in the treatment and prophylaxis of bone metabolic diseases.

SEQUENCE LISTING

<110>  Sankyo Company, Limited

<120>  A complex comprising OCIF and polysaccharide

<130>  EPP85710

<150>  JP 2001-198985

<151>  2001-06-29

<160>  1

<170>  PatentIn version 3.1

<210>  1

<211>  401

<212>  PRT

<213>  Homo sapiens

<220>

<221>  SIGNAL

<222>  (-21)..(-1)

<223>

<220>

<221>  mat_peptide

<222>  (+1)..(+380)

<223>

<400>  1

Met Asn Asn Leu Leu Cys Cys Ala Leu Val Phe Leu Asp Ile Ser Ile
    -20                 -15                 -10

Lys Trp Thr Thr Gln Glu Thr Phe Pro Pro Lys Tyr Leu His Tyr Asp
    -5              -1   1               5                   10

Glu Glu Thr Ser His Gln Leu Leu Cys Asp Lys Cys Pro Pro Gly Thr
                15                  20                  25

Tyr Leu Lys Gln His Cys Thr Ala Lys Trp Lys Thr Val Cys Ala Pro
            30                  35                  40

Cys Pro Asp His Tyr Tyr Thr Asp Ser Trp His Thr Ser Asp Glu Cys
        45                  50                  55

Leu Tyr Cys Ser Pro Val Cys Lys Glu Leu Gln Tyr Val Lys Gln Glu
60                  65                  70                  75

Cys Asn Arg Thr His Asn Arg Val Cys Glu Cys Lys Glu Gly Arg Tyr
                80                  85                  90

Leu Glu Ile Glu Phe Cys Leu Lys His Arg Ser Cys Pro Pro Gly Phe
                95                  100                 105

Gly Val Val Gln Ala Gly Thr Pro Glu Arg Asn Thr Val Cys Lys Arg
        110                 115                 120

Cys Pro Asp Gly Phe Phe Ser Asn Glu Thr Ser Ser Lys Ala Pro Cys
        125                 130                 135

Arg Lys His Thr Asn Cys Ser Val Phe Gly Leu Leu Leu Thr Gln Lys
140                 145                 150                 155

Gly Asn Ala Thr His Asp Asn Ile Cys Ser Gly Asn Ser Glu Ser Thr
                160                 165                 170

Gln Lys Cys Gly Ile Asp Val Thr Leu Cys Glu Glu Ala Phe Phe Arg
                175                 180                 185

Phe Ala Val Pro Thr Lys Phe Thr Pro Asn Trp Leu Ser Val Leu Val
            190                 195                 200

Asp Asn Leu Pro Gly Thr Lys Val Asn Ala Glu Ser Val Glu Arg Ile
        205                 210                 215

Lys Arg Gln His Ser Ser Gln Glu Gln Thr Phe Gln Leu Leu Lys Leu
220                 225                 230                 235

Trp Lys His Gln Asn Lys Asp Gln Asp Ile Val Lys Lys Ile Ile Gln
                240                 245                 250

Asp Ile Asp Leu Cys Glu Asn Ser Val Gln Arg His Ile Gly His Ala
            255                 260                 265

Asn Leu Thr Phe Glu Gln Leu Arg Ser Leu Met Glu Ser Leu Pro Gly
        270                 275                 280

27

```
Lys Lys Val Gly Ala Glu Asp Ile Glu Lys Thr Ile Lys Ala Cys Lys
    285                 290                 295


Pro Ser Asp Gln Ile Leu Lys Leu Leu Ser Leu Trp Arg Ile Lys Asn
300                 305                 310                 315


Gly Asp Gln Asp Thr Leu Lys Gly Leu Met His Ala Leu Lys His Ser
                320                 325                 330


Lys Thr Tyr His Phe Pro Lys Thr Val Thr Gln Ser Leu Lys Lys Thr
                335                 340                 345


Ile Arg Phe Leu His Ser Phe Thr Met Tyr Lys Leu Tyr Gln Lys Leu
    350                 355                 360


Phe Leu Glu Met Ile Gly Asn Gln Val Gln Ser Val Lys Ile Ser Cys
    365                 370                 375


Leu
380
```

**Description**

The present invention relates to a complex comprising at least one osteoclastogenesis inhibitory factor (referred to hereinafter as OCIF), or an analog thereof or a variant thereof and at least one polysaccharide or a derivative thereof, to a method for producing said complex, to a medicament for treating or preventing bone metabolic diseases comprising the complex as an active ingredient, and to the use of said complex in treating or preventing bone metabolic diseases.

Bones contain about 99% of the total calcium present in the living body, and therefore play an important role not only in supporting the body but also functioning as the largest storage organ of calcium in the body. The bones play an important role in maintaining homeostasis of the calcium. It is known that the activation of osteoclasts, which play an important role in bone resorption, causes excessive flow of calcium into the blood from the bones to break the homeostasis of calcium in the blood, thus inducing hypercalcemia. This induction of hypercalcemia by the activation of osteoclasts and promotion of bone resorption can be caused by cytokines that are secreted abnormally as a result of the spread of cancer to the bone [e.g. see Jean-Jacques Body, Current and Future Directions in Medical Therapy: Hypercalcemia, CANCER Supplement, 88(12), 3054-3058 (2000)]. The prognosis for patients suffering from cancerous hypercalcemia is generally poor and it is therefore highly desirable to find an effective treatment for this condition.

In rheumatism such as rheumatoid arthritis and the like or osteoarthritis, the abnormal formation or abnormal activation of osteoclasts is known to be one of the main causes of various of the symptoms that present in the bones and joints of patients suffering from these conditions [e.g. see E. Romas, M. T. Gillespie and T. J. Martin, Involvement of Receptor Activator of NF-κB Ligand and Tumor Necrosis Factor-α in Bone Destruction in Rheumatoid Arthritis, Bone, 30(2), 340-346 (2002)]. The pain in the joints and bones due to rheumatism such as rheumatoid arthritis and osteoarthritis is extremely intense and is seriously deleterious to the quality of life of patients suffering from these conditions. Again, it is therefore highly desirable to find an effective treatment for these conditions.

Osteoclasts are also known to play a role in osteoporosis. The balance of bone resorption promoted by osteoclasts and bone formation promoted by osteobalsts gradually inclines towards bone resorption due to the reduced secretion of female hormones after menopause or due to aging, as a result of which the bone density is lowered and osteoporosis is caused if this reduction in bone density is sufficiently severe. When aged patients with a high risk of osteoporosis suffer a fracture, the possibility that they will become bedridden is high, and this has become a social issue as a result of the increasingly aged population in all parts of the world [e.g. see Bruno Fautrel and Francis Guillemin, Cost of illness studies in rheumatic diseases, Current Opinion in Rheumatology, 14, 121-126 (2002)]. An effective means of treating and preventing osteoporosis is therefore keenly sought after.

Conventional treatments for these conditions include the supplementation of hormones such as estrogen and the use of agents that suppress the activity of osteoclasts such as bisphosphonates or calcitonins [e.g. see Mohammad M. Iqbal and Tanveer Sobhan, Osteoporosis: A Review, Missouri Medicine, 99(1), 19-23 (2002)]. However, hormones can have undesirable side effects such as the raised risk of oncogenesis, the induction of endometriosis and abnormal bleeding from genitals [e.g. see Joyce Penrose White and Judith S. Schilling, Postmenopausal Hormone Replacement: Historical Perspectives and Current Concerns, Clinical Excellence for Nurse Practitioners, 4(5), 277-285 (2000)]. Although it is known that bisphosphonates easily bind excess calcium in the blood and accumulate at bone, some researchers doubt to what extent the strength of bone can be improved thereby. Furthermore, it has also been reported that there is a danger of impaired kidney function associated with their use [e.g. see Jonathan R. Green, Yves Seltenmeyer, Knut A. Jaeggi and Leo Wildler, Renal Tolerability Profile of Novel, Potent Bisphosphonates in Two Short-Term Rat Model, Pharmacology and Toxicology, 80, 225-230 (1997)]. As for calcitonin, the increase in bone density obtained with their use is, unfortunately, transient. It has also been reported that interruption of administration of calcitonin can cause a regression of the condition being treated, while the effectiveness of calcitonins originating from animals other than humans can be reduced after prolonged treatment as a result of the appearance of circulating antibodies to the calcitonin in the human body [S. L. Porcel, J. A. Cumplido, B. dela Hoz, M Cuevas and E. Losada, Anaphylaxis to calcitonin, Allergologia et Immunopathologia, 28(4), 243-245 (2000)].

As explained above, osteoclasts play a major role in promoting bone resorption which is an important factor governing the increase of calcium concentration in the blood. However, it is believed that none of the above-mentioned existing medicines have any activity in suppressing the formation of osteoclasts. Consequently, none of these conventional medicines is suitable for fundamental treatment of bone metabolic diseases as they are only able to manage the symptoms rather than address the causes.

More recently, OCIF has been demonstrated to be an endogenic protein which inhibits differentiation of an osteoclast precursor cell to an osteoclast and/or the bone resorption activity of the mature osteoclast (see WO-A-96/26217 and EP-A-0816380). OCIF is also known as osteoprotegerin (see WO-A-97/23614). In view of the fact that the abovementioned bone metabolic diseases such as hypercalcemia, osteoporosis and rheumatoid arthritis all result at least to some extent from bone resorption, it was hoped that these diseases could be successfully treated using OCIF due to this ability to suppress the formation of the osteoclast itself and/or to suppress the bone resorption activity of the mature osteoclast. However, OCIF is a basic protein which has an isoelectric point of around 9, and it disappears very rapidly from the bloodstream after administration. An attempt to address this problem is disclosed in WO-A-2000/24416 and EP-A-1127578 where the length of time that OCIF remains in the blood after administration (and hence the effect of the OCIF) was prolonged to a certain extent by co-administering the OCIF with a polysaccharide such as heparin or dextran sulfate. However, the prolongation of the retention time achieved as a result may not be sufficient to give the sort of prolonged retention of OCIF in the blood that would make it a genuine candidate for use in the treatment of bone metabolic diseases such as hypercalcemia, osteoporosis and rheumatism. There is, therefore, a need for an improved means of prolonging the length of time that OCIF is retained in the bloodstream after administration.

It is therefore an object of the present invention to provide a preparation comprising OCIF which enables the length of time that OCIF is retained in the bloodstream after administration to be prolonged, thus providing an agent in which the effect of OCIF in the treatment and prophylaxis of bone metabolic diseases which are mediated by osteoclasts, such as hypercalcemia, osteoporosis and rheumatism, is enhanced and prolonged.

Other objects and advantages of the present invention will become apparent as the description proceeds.

Thus, the present invention provides a complex comprising at least one substance selected from the group consisting of OCIF, analogues thereof and variants thereof, which is bound to at least one substance selected from the group consisting of polysaccharides and derivatives thereof.

The present invention also provides a method for prolonging the time that OCIF or an analogue or variant thereof is retained in the bloodstream after administration to a patient by complexing at least one of said OCIF, said analogue thereof or said variant thereof with at least one polysaccharide or a variant thereof.

The present invention also provides a pharmaceutical composition comprising an effective amount of a pharmacologiocally active agent together with a carrier or diluent therefor, wherein said pharmacologiocally active agent is a complex comprising at least one substance selected from the group consisting of OCIF, analogues thereof and variants thereof, which is bound to at least one substance selected from the group consisting of polysaccharides and derivatives thereof. In particular, it provides such a pharmaceutical composition for the treatment or prophylaxis of bone metabolic diseases.

The present invention also provides the use of a complex comprising at least one substance selected from the group consisting of OCIF, analogues thereof and variants thereof, which is bound to at least one substance selected from the group consisting of polysaccharides and derivatives thereof in the manufacture of a medicament for the prophylaxis or treatment of bone metabolic diseases.

We have found that by incubating at least one substance selected from OCIF, analogues and variants thereof with at least one substance selected from polysaccharides and derivatives thereof under conditions that result in the formation of a complex in which said one or more substances selected from OCIF, analogues and variants thereof are bound to said at least one substance selected from polysaccharides and derivatives thereof, an agent is thereby produced in which the effect of said OCIF or analogue or variant thereof in the treatment and prophylaxis of bone metabolic diseases which are mediated by osteoclasts, such as hypercalcemia, osteoporosis and rheumatism, is enhanced and prolonged. This is due to the fact that the length of time that said OCIF or analogue or variant thereof is retained in the bloodstream after administration is prolonged when compared to the prior art combinations of OCIF and polysaccharides disclosed in WO-A-2000/24416 and EP-A-1127578.

As noted above, the complexes of the present invention comprise at least one substance selected from OCIF, analogues and variants thereof which are bound to at least one substance selected from polysaccharides and derivatives thereof. In said complex, the OCIF and polysaccharide are bound to each other by a chemical bond such as a covalent bond (e.g. Schiff base formation), an ionic bond or a coordinate bond, or by a non-chemical bond such as a hydrophobic interaction, a hydrogen bond, an electrostatic interaction or affinity binding.

OCIF, an analogue thereof or a variant thereof used in the present invention can be natural type or it can be recombinant type and its origin is not particularly limited. Natural type OCIF means OCIF that is obtained as a naturally produced protein by extraction, purification and/or isolation from an organ, a body fluid, a cell culture, or a culture medium derived from a human or a non-human animal. Recombinant type OCIF, an analogue thereof or a variant thereof is a recombinant protein obtained by extraction, purification and/or isolation of said protein from a host conventionally used in such techniques such as a prokaryotic host cell (e.g. *Escherichia coli*) or a eukaryotic cell such as a human or a non-human cell line which has been transformed with a vector comprising a polynucleotide which encodes an OCIF, an analogue thereof or a variant thereof [e.g. see the recombinant methods disclosed in EP-A-0816380 (WO-A-96/26217) and WO-A-97/23614].

The origin of the OCIF, analogues thereof and variants thereof used in the present invention is not particularly limited and they can be derived from a human or a non-human animal. Preferably, they can be derived from a mammal such as a human, rat, mouse, rabbit, dog, cat, cow, swine, sheep or goat; or an avian such as a fowl, goose, chicken or turkey. More preferably, they are derived from mammals and most preferably they are derived from a human.

The OCIF or analogue thereof used in the present invention can be a monomer-type OCIF (e.g. in humans a monomer having a molecular weight as measured by SDS-PAGE under non-reducing conditions of about 60000) or a dimer type (e.g. in humans a dimer having a molecular weight of about 120000 as measured by SDS-PAGE under non-reducing conditions) [see EP-A-0816380 (WO-A-96/26217)].

It is known that OCIF is translated in cells as a polypeptide containing a signal peptide at the amino terminus thereof and that it is then matured by processing involving the removal of said signal peptide [e.g. see the recombinant methods disclosed in EP-A-0816380 (WO-A-96/26217) and WO-A-97/23614]. The OCIF, analogue thereof or variant thereof used in the present invention includes both the polypeptide containing a signal peptide and the matured form thereof. Preferred examples include the OCIF with the signal peptide having amino acids -21 to +380 of SEQ. ID. NO. 1 of the sequence listing and the mature OCIF without the signal peptide having amino acids +1 to +380 of SEQ. ID. NO.1 of the sequence listing. Of these, the mature OCIF is particularly preferred.

It is also known that methionine can be added to such a matured form of OCIF, an analogue thereof or a variant thereof, when it is expressed as a recombinant protein in a host cell, especially in a prokaryotic host cell such as *Escherichia coli*. This is achieved by adding a nucleotide triplet having the sequence ATG (AUG) to the 5'-end of a polynucleotide encoding a matured form of OCIF, an analogue thereof or a variant thereof, and inserting the resultant polynucleotide into a suitable expression vector. The desired matured protein having methionine at the amino terminus thereof can be then expressed by a suitable host cell which has been transformed by said recombinant expression vector. Additionally, one or more than one amino acid can be added to said protein at a position next to the amino terminal methionine by the addition of further nucleotide triplets next to the ATG triplet added at the 5'-end of the polynucleotide encoding a matured form of OCIF, an analog thereof or a variant thereof.

In the present invention, an OCIF analogue means a protein encoded by a polynucleotide which exists naturally in the cells, body fluid, and/or organs of a human or non-human animal such as those exemplified above. Specific preferred examples of such analogues include OCIF2, OCIF3, OCIF4 and OCIF5 [see EP-A-0816380 (WO96/26217)]. Such OCIF analogues or active fragments thereof can be obtained by a method such as the following: RNA is extracted from a cell, organ, tissue or body fluid of a human or non-human animal; a first strand of cDNA which is complementary to said RNA is synthesized using a reverse transcriptase, and then a second strand of said cDNA is synthesized using the first as a template using a DNA polymerase; the double-stranded cDNA thus-obtained is inserted into a suitable, conventionally-used expression vector; a suitable, conventionally-used host cell is then transformed by the vector thus-obtained; the host producing the desired peptide is then screened for using a hybridization technique such as plaque hybridization or phage hybridization using OCIF cDNA or a fragment thereof as a probe under stringent conditions [see EP-A-0816380 (WO-A-96/26217)]; and then finally the desired OCIF analogue is expressed by a conventional technique by the thus-obtained host cell.

In the present invention, an OCIF variant means a protein which has an amino acid sequence wherein one or more than one amino acid residues have been substituted in, deleted from, added to or inserted in the amino acid sequence of an OCIF or an analogue thereof, and still has at least some OCIF activity. Such OCIF variants can be obtained by, for example, the following method: substituting, deleting, adding and/or inserting one nucleotide or more than one nucleotides in a nucleotide sequence encoding OCIF or an analogue thereof using a polymerase chain reaction method (referred to hereinafter as PCR), a genetic recombination method or a nuclease digestion method using an exonuclease or endonuclease such as a restriction enzyme; transforming a eukaryotic host cell such as an animal cell or a prokaryotic host cell such as *Escherichia coli* with an expression vector wherein the obtained nucleotide encoding the desired OCIF variant has been inserted; and then extracting, purifying and/or isolating the desired pepetide from the protein-containing fraction produced by a cell culture of said transformed host according to a method well-known to the person skilled in the art.

Truncated forms of OCIF wherein a considerable part of the amino acid sequence has been deleted from the carboxy terminus of an OCIF polypepetide are also known to keep at least some OCIF activity [e.g. see EP-A-0816380 (WO-A-96/26217) and WO-A-97/23614]. Such truncated types of OCIF retaining at least some of the activity of the complete OCIF polypeptide are also included in the OCIF variants of the present invention.

Furthermore, OCIF or a truncated form thereof that is fused with the an immunoglobulin domain such as the Fc domain (e.g. a fusion polypeptide in which the Fc domain of human IgG is attached to the carboxy terminus of OCIF) and which retains at least some of the activity of the complete OCIF polypeptide is known (see WO-A-97/23614), and such fusion proteins are also included in the OCIF variants of the present invention.

It has also been shown that OCIF or an analogue thereof or a variant thereof can be chemically modified and still retain useful activity and, in some cases, may show advantages such as increased stability or decreased immunogenicity. Such chemical modification can involve derivatization at just a single site in the molecule of the OCIF or an analogue thereof or a variant thereof or at more than one site. For example, it has been shown that OCIF and variants (derivatives) thereof such as a truncated form can be chemically modified with one or more water soluble polymers such as polyethylene glycol, ethylene glycol/propylene glycol copolymers, carboxymethylcellulose and polyvinylalcohol, and can show improved biological activity as a result (e.g. see WO-A-97/23614). Such chemically modified types of OCIF or an analogue thereof or a variant thereof are also included in the OCIF variants of the present invention.

Examples of known OCIF variants that are suitable for use in preparation of the complexes of the present invention include: OCIF-C19S, OCIF-C20S, OCIF-C21S, OCIF-C22S, OCIF-C23S, OCIF-DCR1, OCIF-DCR2, OCIF-DCR3, OCIF-DCR4, OCIF-DDD1, OCIF-DDD2, OCIF-CL, OCIF-CC, OCIF-CDD2, OCIF-CDD1, OCIF-CCR4, OCIF-CCR3, OCIF-CBst, OCIF-CSph, OCIF-CBsp, OCIF-CPst [see EP-A-0816380 (WO-A-96/26217)], muOPG [22-401]-Fc, muOPG[22-194]-Fc, muOPG[22-185]-Fc, muOPG[22-180]-Fc, muOPG[22-401], muOPG[22-401]C195, muOPG[22-401]C202, muOPG[22-401]C277, muOPG[22-401]C319, muOPG[22-401]C400, muOPG[22-185], muOPG[22-194], muOPG[22-200], muOPG[22-212], muOPG[22-293], muOPG[22-355], huOPG[22-401]-Fc, huOPG [22-201]-Fc, huOPG[22-401]-Fc P26A, huOPG[22-401]-Fc Y28F, huOPG[22-401], huOPG[27-401]-Fc, huOPG [29-401]-Fc, huOPG[32-401]-Fc, muOPG met[22-194], muOPG met[22-194] 5k PEG, muOPG met[22-194] 20k PEG, huOPG met[22-194]P25A, huOPG met[22-194]P25A 5k PEG, huOPG met[22-194]P25A 20k PEG, huOPG met [22-194]P25A 31k PEG, huOPG met[22-194]P25A 57k PEG, huOPG met[22-194]P25A 12k PEG, huOPG met[22-194] P25A 20k Branched PEG, huOPG met[22-194]P25A 8k PEG dimer, huOPG met[22-194] P25A disulfide crosslink (WO-A-97/23614), OPG[22-194]-Fc, OPG[22-201]-Fc, OPG[22-194]-Fc $\Delta$ C, OPG[22-201]-Fc $\Delta$ C, OPG[22-194] -FcG$_{10}$, metFc $\Delta$ C-OPG[22-194] (WO-A-2001/17543), OPG[22-194]-Fc $\Delta$ C, OPG[22-194]-FcG$_{10}$, Fc $\Delta$ C-OPG [22-194], metFc $\Delta$ C-OPG[22-194], metFc $\Delta$ C-22-194, OPG[22-194]-Fc, OPG[22-194]-Fc $\Delta$ C, metOPG[22-194], metOPG[22-201], OPG[22-293], OPG[22-401] and metFc $\Delta$ C-22-194 (WO-A-2001/18203).

Of these, preferred examples include: OCIF-C19S, OCIF-C20S, OCIF-C21S, OCIF-C22S, OCIF-C23S, OCIF-DCR1, OCIF-DCR2, OCIF-DCR3, OCIF-DCR4, OCIF-DDD1, OCIF-DDD2, OCIF-CL, OCIF-CC, OCIF-CDD2, OCIF-CDD1, OCIF-CCR4, OCIF-CCR3, OCIF-CBst, OCIF-CSph, OCIF-CBsp, OCIF-CPst, muOPG[22-401]-Fc, muOPG[22-194]-Fc, muOPG[22-185]-Fc, muOPG[22-401]C195, muOPG[22-401]C202, muOPG[22-401]C319, muOPG[22-401]C400, muOPG[22-194], muOPG[22-200], muOPG[22-293], muOPG[22-355], huOPG[22-401]-Fc, huOPG[22-201]-Fc, huOPG[22-401]-Fc P26A, huOPG[22-401]-Fc Y28F, huOPG[22-401], huOPG[27-401]-Fc, huOPG [29-401]-Fc, huOPG[32-401]-Fc, muOPG met[22-194]5k PEG, muOPG met[22-194]20k PEG, huOPG met[22-194] P25A 5k PEG, huOPG met[22-194]P25A 20k PEG, huOPG met[22-194]P25A 31k PEG, huOPG met[22-194]P25A 57k PEG, huOPG met[22-194]P25A 12k PEG, huOPG met[22-194]P25A 20k Branched PEG, huOPG met[22-194] P25A 8k PEG dimer, huOPG met[22-194]P25A disulfide crosslink, OPG[22-194]-Fc, OPG[22-201]-Fc, OPG[22-194] -Fc $\Delta$ C, OPG[22-201]-Fc $\Delta$ C, OPG[22-194]-FcG$_{10}$, metFc $\Delta$ C-OPG[22-194], OPG[22-194]-Fc $\Delta$ C, OPG[22-194] -FcG$_{10}$, Fc $\Delta$ C-OPG[22-194], metFc $\Delta$ C-OPG[22-194], metFc $\Delta$ C-22-194, OPG[22-194]-Fc, OPG[22-194]-Fc $\Delta$ C, metOPG[22-194], metOPG[22-201], OPG[22-293], OPG[22-401] and metFc $\Delta$C-22-194.

OCIF or an analogue or variant thereof of the present invention can contain a sugar chain as part of the molecule. Any naturally-produced OCIF or an analogue thereof or recombinant OCIF or analogue or variant thereof can contain a sugar chain which is attached to the OCIF or analogue or variant thereof post-translationally. Natually-produced OCIF or an analogue thereof containing a sugar chain can be obtained from cell cultures, tissues, organs, body fluids or cell lines derived from human or non-human animals using conventional techniques. Recombinant OCIF or an analogue or variant thereof containing a sugar chain can be obtained from a culture of a eukaryotic host cell transformed using a vector comprising a nucleotide sequence encoding any OCIF or an analogue or variant thereof such as those described and exemplified above. Examples of suitable host cells that can be used which are capable of the post-translational modification of OCIF or an analogue or variant thereof so as to attach a sugar chain include chinese hamster ovary cells and COS cells [Yasuda, H. et al, Endocrinology, 139, 1329-1337 (1998)]. OCIF or an analogue or variant thereof containing such a sugar chain is suitable for use in the formation of the complexes of the present invention.

If, on the other hand, it is desired to produce a recombinant OCIF or an analogue or variant thereof that does not have a sugar chain that has been added as a post-translational modification, then the preferred host cells are prokaryotic cells such as *Escherichia coli*.

The polysaccharide used in the formation of the complexes of the present invention is a polymer (glycan) produced by the glycosidic linkage of two or more monosaccharides, and is preferably a heteropolysaccharide (heteroglycan) consisting of at least two different kinds of monosaccharide. Any polysaccharide, whether naturally-occurring or synthetic can potentially be used in the complex of the present invention.

In the present invention, a derivative of a polysaccharide is a polysaccharide wherein at least a part of said polysaccharide molecule is substituted by one or more than one molecules and/or residues other than a saccharide or sugar. Preferred derivatives include acid esters of polysaccharides, and particularly preferred are sulfate esters of polysaccharides.

Examples of natural polysaccharides suitable for use in the formation of the complexes of the present invention include hyaluronic acid, chondroitin sulfuric acid, dermatan acid, heparan acid, keratan acid, carrageenan, pectin and heparin. Examples of synthetic polysaccharides suitable for use in the formation of the complexes of the present invention include dextran while examples of suitable synthetic polysaccharide derivatives include dextran sulfate. Of the polysaccharides and derivatives thereof, the most preferred for use in the formation of the complexes of the present invention is dextran sulfate.

In the present invention, polysaccharides and derivatives thereof such as dextran sulfate include salts thereof. The most preferred salt of dextran sulfate is the sodium salt thereof. Examples of sodium salts of dextran sulfate include dextran sulfate sodium salt sulfur 5 (referred to hereinafter as DS5: manufactured by Meito Sangyo Co., Ltd.), and dextran sulfate sodium salt 5000 and dextran sulfate sodium salt 10000 (both of them are manufactured by Wako Pure Chemical Industries, Ltd.).

The molecular weight of a dextran sulfate is calculated as follows.

1) Measurement of the molecular weight of dextran

The molecular weight of dextran can be calculated according to Sato's formulation shown below [e.g. see Manual for Pharmacopoeia of Japan, the thirteenth revision, published by Hirokawashoten (1998), the entry concerning dextran 40] based on the measurement of the limiting viscosity of said dextran.

$$\text{Limiting viscosity} = 9.00 \times 10^{-4} \times \text{molecular weight}^{0.50}$$

2) Measurement of sulfur content

The sulfur content in the dextran sulfate of interest can be measured as a weight % by any conventional technique known in the art, e.g. the method described in the entry concerning dextran sulfate sodium salt sulfur 5 in Pharmacopoeia of Japan [14th revision, published by Jihou (2001)].

While the molecular weight of glucose, which is a unit of dextran, is 180, the actual molecular weight of the glucose unit in a dextran molecule is 162, this value being obtain by subtracting the molecular weight of water from 180 because adjacent glucose units are bound to each other by an $\alpha$-1,6 glycosidic linkage in the dextran molecule. A hydrogen atom is replaced by a sodium sulfate group ($SO_3Na$: one gram equivalent = 103) in each glucose unit of a dextran sulfate that is substituted in this manner. Using this information, the degree of substitution of a dextran sulfate molecule (hereinafter referred to as the "substitution degree") can be determined from the following formula:

$$\text{Sulfur content (weight \%)} = [32 \times \text{substitution degree} / (162 + 102 \times \text{substitution degree})] \times 100$$

3) Calculation of the molecular weight of a dextran sulfate

Since, as noted above, the actual molecular weight of the glucose unit in the dextran chain is 162, the molecular weight of a dextran sulfate can be calculated from this information and the degree of substitution determined in (2) above using the following formula:

$$\text{Molecular weight of dextran sulfate} = \text{molecular weight of dextran} \times (162 + 102 \times \text{substitution degree})/162$$

It is known that polysaccharides display a distribution of molecular weights, e.g. each different type of dextran sulfate displays a certain molecular weight distribution. The molecular weight of any polysaccharide used in formation of the complexes of the present invention is given as an average molecular weight. The average molecular weight of the polysaccharides used in the present invention is not limited in any way. The range of the average molecular weight of the most preferred polysaccharide derivative of the present invention, dextran sulfate is generally 1500 to 12000, and is more preferably 1800 to 6000. The molecular weight (average $\pm$ standard deviation) of DSS is about $1950 \pm 70$ (n = 7). The sulfur substitution degree (average $\pm$ standard deviation) of DS5, calculated as described above, is about $0.32 \pm 0.01$ (n = 7). The average molecular weight of dextran sulfate sodium salt 5000 and dextran sulfate sodium salt 10000 are about 5000 and about 10,000, respectively. The polysaccharides used in preparation of the complexes of the present invention may be used without or with any further purification and/or fractionation therefrom before use. In the present invention, polysaccharides or derivatives thereof do not include any sugar chain which is attached to recombinant OCIF or analogues or variants thereof or to naturally-produced OCIF or analogues or variants thereof post-translationally and/or endogenously in cells or tissues or bodies of human or non-human animals.

The molecular ratio of the substance selected from the group consisting of OCIF, analogues thereof and variants thereof to the substance selected from the group consisting of polysaccharides and derivatives thereof in the complexes of the present invention will vary depending upon various factors including the identity of the components of said complex and the conditions under which the complex is prepared. There is no particular limitation on the molecular ratio of the substance selected from the group consisting of OCIF, analogues thereof and variants thereof to the substance selected from the group consisting of polysaccharides and derivatives thereof in the complexes of the present invention. In the preferred complexes of the present invention comprising a substance selected from the group consisting of OCIF, analogues thereof and variants thereof and dextran sulfate, the molecular ratio of said substance selected from the group consisting of OCIF, analogues thereof and variants thereof : dextran sulfate is from 1:1 to 1: 10; more preferably the molecular ratio is from 1:1 to 1:8; yet more preferably the molecular ratio is from 1:1 to 1:5; and most preferably the molecular ratio is from 1:1.1 to 1:4.5.

As has already been mentioned above, OCIF or an analogue or variant thereof can exist as a monomer or can form dimers, such that OCIF or an analogue or variant thereof present in the complexes of the present invention can be a homodimer or a heterodimer, or it can be a homooligomer, heterooligomer, homopolymer or heteropolymer comprising more than two monomeric units of OCIF, an analogue thereof or a variant thereof (e.g. see US 6,369,027). The molecular ratio of the substance selected from the group consisting of OCIF, analogues thereof and variants thereof to the substance selected from the group consisting of polysaccharides and derivatives thereof in a complex comprising OCIF, or an anlogue or variant thereof and polysaccharides or a derivative thereof according to the present invention is calculated as the number of molecules of polysaccharide or derivative thereof per monomeric unit of OCIF, variant thereof or analogue thereof.

The number of molecules of polysaccharide or derivative thereof in a complex of the present invention can preferably be determined as follows. The neutral sugar content of the tested complex [designated as (x)] and that of a reference sample that contains only the uncomplexed, free OCIF or analogue or variant thereof [designated as (y)] are quantified using the phenol sulfuric acid method (which is described in detail elsewhere in the present application). The amount of polysaccharide or derivative thereof which is bound to OCIF or an analogue or variant thereof in the tested complex is then determined by subtracting (y) from (x). Using the figure thus obtained, the number of molecules of polysaccharide or derivative thereof which are bound to OCIF or an analogue or variant thereof is calculated according to (I) or (II) below:

(I) The obtained figure for the amount of polysaccharide or derivative thereof which is bound to OCIF or an analogue or variant thereof is divided by the average molecular weight of said polysaccharide or derivative thereof. The resultant figure represents the total number of molecules of polysaccharide or derivative thereof in the test complex.
(II) The obtained figure for the amount of polysaccharide or derivative thereof which is bound to OCIF or an analogue or variant thereof is divided by the amount (mg) of said OCIF, analogue or variant thereof in said complex. The resulting figure, which is the amount of polysaccharide or derivative thereof per 1 mg of OCIF, analogue or variant thereof in the complex, is then used to calculate the number of molecules of polysaccharide or derivative thereof per one molecule of OCIF, analogue or variant thereof on the basis of the average molecular weight of said polysaccharide or derivative thereof and the molecular weight of said OCIF, analogue or variant thereof [e.g. according to Example 4(d) below].

The number of molecules of OCIF or an analogue or variant thereof in a complex of the invention can preferably be determined using an immunological assay technique, such as those described elsewhere in the present application.

A preferred feature of the complexes of the present invention that can be used to characterise them is their affinity to heparin. Heparin is a polysaccharide comprising D-glucosamine, D-glucuronic acid and D-iduronic acid which is partially or fully derivatised with sulfate and acetyl groups. A preferred feature of the complexes of the present invention is that the strength of adsorption of said complex of OCIF or an anlogue or variant thereof to heparin is lower than the strength of adsorption of the free, non-complexed OCIF or analogue or variant thereof. The degree of adsorption can be determined using a column packed with highly cross-linked agarose beads on which has been immobilized heparin (e.g. heparin obtained from bovine intestinal mucosa). Suitable columns of this type include HiTrap heparin HP column, HiPrep 16/10 Heparin and Heparin Sepharose (all obtainable from Amersham Pharmacia). The strength of adsorption (the affinity) of the complex can be determined according to any suitable method that is well known to the person skilled in the art for determining the affinity of proteins to polysaccharides. Preferably, the degree of adsorption can be determined by comparing the amount of the complex that binds to the heparin column under low ionic strength conditions but that is eluted from said column under high ionic strength conditions with the amount of complex that does not bind to the heparin column under low ionic strength conditions (the ionic strength can be adjusted using the salt of a strong acid such as sodium chloride). Thus, typically the degree of adsorption of the complex to heparin can be determined as follows:

(a) A column packed with a support such as cross-linked agarose beads on which has been immobilized heparin is equilibrated with a buffer having a relatively low ionic strength (e.g. sodium phosphate buffer containing 0.1-0.8 M sodium chloride).
(b) The complex of the present invention that is being tested is dissolved in the same low ionic strength buffer as used in (a) and applied to the column and a first eluate is then collected (fraction A).
(c) The column is then washed further with the same low ionic strength buffer as used in step (a) and a second eluate is collected (fraction B).

(d) The column is then washed with a buffer having a relatively high ionic strength (e.g. sodium phosphate buffer containing 1.0-2.0 M sodium chloride) and a third eluate is then collected (fraction C).

(e) The amount of the complex present in each of fractions A, B and C [designated (a), (b) and (c) respectively] is then determined (e.g. by an immunoassay).

(f) The degree of adsorption of the complex to heparin is then determined according to the following formula:

$$\text{degree of adsorption} = \frac{(c)}{(a) + (b) + (c)}$$

The greater the strength of the binding of the complex to the column, the higher is the value of (c) (as it can only be removed from the column using eluants having a relatively high ionic strength) and hence the higher is the degree of adsorption. The degree of adsorption of the complexes of the present invention as measured by the above formula will vary to some extent depending upon the type of heparin column and the conditions under which the determination is carried out. However, the degree of adsorption of free, uncomplexed OCIF is always around 1.0 whereas the degree of adsorption of the complexes of OCIF of the present invention is less than 1.0, thus demonstrating that the strength of binding of the complexes comprising OCIF or an analogue or variant thereof of the present invention to heparin is weaker than the strength of binding of the free, uncomplexed OCIF or analogue or variant thereof (e.g. using porcine heparin immobilized on agarose beads, such as a HiTrap heparin HP column, first and second elutions with 10 mM sodium phosphate buffer containing 0.7 M sodium chloride and a third elution with 10 mM sodium phosphate buffer containing 2.0 M sodium chloride, the degree of adsorption of complexes of the present invention comprising OCIF or a variant thereof or an analogue thereof is not greater than 0.7, preferably not greater than 0.6 and particularly preferably not greater than 0.5).

Another preferred feature of the complexes of the present invention that can be used to characterise them is the ratio of the number of molecules of OCIF or an analogue or variant thereof present in said complex as measured by an immunological assay technique (e.g. ELISA) to the number of molecules of OCIF or an analogue or variant thereof present in said complex as measured by a technique for measuring the total amount of protein present in said complex [e.g. Lowry's method: Lowry, O.H. *et al*, J. Biol. Chem, **193**, 265-275 (1951), absorbance ($\lambda$ 280 nm) silver staining or the BCA method].

The number of molecules of OCIF or an analogue or variant thereof present in said complex as measured by an immunological assay technique can be determined using, for example, ELISA. The antibodies for use in binding to the immobilized phase and for labeling with a reporter enzyme such as a peroxidase in ELISA are any antibodies to the OCIF or analogue or variant thereof of interest that are suitable for the purpose. For example, suitable antibodies for binding to the solid phase include OI-26 purified from a culture of a hybridoma producing antibody OI-26 (FERM BP-6421) and OI-19 purified from a culture of a hybridoma producing antibody OI-19 (FERM BP-6420), while suitable antibodies for use as the antibody labeled with a reporter enzyme in the mobile phase include anti-human OCIF monoclonal antibody OI-4 purified from a culture of a hybridoma producing antibody OI-4 (FERM BP-6419) labeled with peroxidase. A typical procedure for measuring the number of molecules of OCIF or an analogue or variant thereof in a complex is as follows:

(a) Known concentrations of the free, uncomplexed OCIF are used to produce a calibration curve.
(b) An ELISA is performed on the complex of interest and the calibration curve is then used to determine the concentration of OCIF.
(c) Using the information obtained in (b) and the molecular weight of the OCIF monomer the number of molecules of OCIF in the tested complex is calculated.

The number of molecules of OCIF or an analogue or variant thereof present in said complex as measured by a technique for measuring the total amount of protein present in said complex can be determined using, for example Lowry's method. A typical procedure is as follows:

(a) Known concentrations of bovine serum albumin are used to produce a calibration curve.
(b) Lowry's method is then used to determine the total concentration of protein in the complex to be tested, the calibration curve being used to determine the concentration of OCIF.
(c) Using the information obtained in (b) and the molecular weight of the OCIF monomer, the number of molecules of OCIF in the tested complex is calculated.

The actual ratio varies depending upon the type of immunoassay technique used and/or the technique used to measure the total protein. A preferred embodiment of the present invention comprises a complex of a human-originating OCIF or an analogue or variant thereof with dextran sulfate, wherein the ratio of the number of molecules of said OCIF or analogue or variant thereof present in said complex as determined by enzyme-linked immunosorbent assay (ELISA) using anti-human OCIF monoclonal antibody OI-19 purified from a culture of a hybridoma producing antibody OI-19 (FERM BP-6420) as the antibody bound to the solid phase and anti-human OCIF monoclonal antibody OI-4 purified from a culture of a hybridoma producing antibody OI-4 (FERM BP-6419) labeled with peroxidase in the mobile phase to the number of molecules of OCIF or analogue or variant thereof present in said complex as determined by measuring the total protein content using Lowry's method is at least 0.5 but not greater than 1.2. More preferably, the ratio is at least 0.6 but not more than 1.1, and most preferably the ratio is at least 0.7 but not more than 1.1.

Preferred complexes of the present invention include the following:

(a) a complex wherein said substance selected from the group consisting of OCIF, analogues thereof and variants thereof is human monomeric OCIF having a molecular weight as measured by SDS-PAGE under non-reducing conditions of about 60000 or human dimeric OCIF having a molecular weight of about 120000 as measured by SDS-PAGE under non-reducing conditions and said polysaccharides and derivatives thereof are selected from the group consisting of hyaluronic acid, chondroitin sulfuric acid, dermatan acid, heparan acid, keratan acid, carrageenan, pectin, heparin, dextran and derivatives thereof, the molecular ratio of said substance selected from the group consisting of OCIF, analogues thereof and variants thereof to said substance selected from the group consisting of polysaccharides and derivatives thereof being from 1:1 to 1:10;

(b) a complex wherein said substance selected from the group consisting of OCIF, analogues thereof and variants thereof is human monomeric OCIF having a molecular weight as measured by SDS-PAGE under non-reducing conditions of about 60000 or human dimeric OCIF having a molecular weight of about 120000 as measured by SDS-PAGE under non-reducing conditions and said polysaccharides and derivatives thereof are selected from the group consisting of dextran sulfate and salts thereof, the molecular ratio of said substance selected from the group consisting of OCIF, analogues thereof and variants thereof to said substance selected from the group consisting of polysaccharides and derivatives thereof being from 1:1 to 1:10;

(c) a complex wherein said substance selected from the group consisting of OCIF, analogues thereof and variants thereof is human monomeric or dimeric OCIF in which said monomer or one of the units of said OCIF dimer comprises amino acids +1 to +380 of SEQ. ID. NO.1 of the sequence listing and said polysaccharide derivative is a sodium salt of dextran sulfate having an average molecular weight of from 1500 to 12000, the molecular ratio of said substance selected from the group consisting of OCIF, analogues thereof and variants thereof to said sodium salt of dextran sulfate being from 1:1 to 1:10;

(d) a complex according to (c) wherein the molecular ratio of said substance selected from the group consisting of OCIF, analogues thereof and variants thereof to said sodium salt of dextran sulfate being from 1:1 to 1:8;

(e) a complex according to (c) wherein the molecular ratio of said substance selected from the group consisting of OCIF, analogues thereof and variants thereof to said sodium salt of dextran sulfate being from 1:1 to 1:5; and

(f) a complex according to any one of (c) to (e) wherein said polysaccharide derivative is a sodium salt of dextran sulfate having an average molecular weight of from 1800 to 6000.

The complexes of the present invention can be prepared using any suitable method that favours binding of the polysaccharide or variant thereof to the OCIF or analogue or variant thereof. In a further embodiment of the present invention, there is provided a method for the preparation of a complex comprising at least one substance selected from the group consisting of OCIF, analogues thereof and variants thereof, which is bound to at least one substance selected from the group consisting of polysaccharides and derivatives thereof, said method comprising the steps of incubating said at least one substance selected from the group consisting of OCIF, analogues thereof and variants thereof with said at least one substance selected from the group consisting of polysaccharides and derivatives thereof under conditions favouring the formation of a complex between said OCIF, analogues thereof or variants thereof and said polysaccharides or variants thereof and then removing any free polysaccharides or variants thereof that are not bound to said OCIF, analogues thereof or variants thereof.

The incubation of said at least one substance selected from the group consisting of OCIF, analogues thereof and variants thereof with said at least one substance selected from the group consisting of polysaccharides and derivatives thereof is performed under any suitable conditions, but typically the incubation takes place under aqueous conditions. Preferably, the incubation is performed under alkaline conditions. More preferably, the incubation is performed at a pH of from 9.5 to 12. Most preferably, the incubation is performed at a pH of from 10 to 11.

During incubation, the range of the concentration of said OCIF, analogue or variant thereof in the aqueous solution is not particularly limited, as long as it is suitable to enable formation of the desired complex. Typically, the maximum concentration of said OCIF, analogue or variant thereof in the aqueous solution is from 0.1 to 0.5 mM and the minimum concentration is from 0.001 to 0.05 mM. Preferably, the concentration of said OCIF, analogue or variant thereof in the aqueous solution is from 0.01 to 0.2 mM, and most preferably it is from 0.05 to 0.1 mM. In the case of OCIF, the maximum concentration in the aqueous solution is from 10 to 50 mg/ml and the minimum concentration is from 0.1 to 5 mg/ml. Preferably, the concentration of OCIF in the aqueous solution is from 1 to 20 mg/ml, and more preferably it is from 5 to 10 mg/ml.

During incubation, the range of the concentration of said polysaccharide or variant thereof in the aqueous solution is not particularly limited, as long as it is suitable to enable formation of the desired complex. Typically, the maximum concentration of said polysaccharide or derivative thereof in the aqueous solution is from 0.1 to 0.5 M and the minimum concentration is from 0.00005 to 0.05 M. Preferably, the concentration of said polysaccharide or derivative thereof in the aqueous solution is from 0.005 to 0.25 M, and more preferably it is from 0.05 to 0.1 M. In the case of dextran sulfate sodium salt sulfur 5, the maximum concentration of said polysaccharide or variant thereof in the aqueous solution is from 200 mg/ml to 1000 mg/ml, and the minimum concentration is from 0.1 to 100 mg/ml Preferably, the concentration of said polysaccharide or variant thereof in the aqueous solution is from 10 to 500 mg/ml and most preferably it is from 100 to 200 mg/ml.

During incubation, the temperature is not particularly limited, as long as it is suitable to enable formation of the desired complex. Typically, the upper limit of temperature for the incubation is from 10 to 50 °C, and the lower limit thereof is from 0 to 4 °C. Preferably, the temperature range is from 4 to 37 °C, and most preferably the temperature range is from 4 to 10 °C.

As noted above, the complex of the present invention does not comprise free polysaccharides or variants thereof which are not bound to OCIF, or an analogue or variant thereof. The method used to remove the free polysaccharides and variants thereof is not limited, as long as it is a method that is conventionally employed in procedures such as purification, isolation and/or fractionation. Examples of suitable methods include ion exchange chromatography, adsorption chromatography, partition chromatography, gel filtration chromatography, hydrophobic chromatography, affinity chromatography, crystallization, salting out and ultrafiltration. Of these, gel filtration chromatography (hereinafter referred to as "gel filtration") and ultrafiltration are preferred and gel filtration is most preferred.

There is no particular limitation on the gel used for the gel filtration for removal of free polysaccharides or variants thereof from the desired complex after incubation as long as it can be used for separation of the fraction containing the desired complex from the free polysaccharide or variants thereof which are not bound to the OCIF. Suitable examples include agarose gel, dextran gel and polyacrylamide gel.

The complexes of the present invention comprising at least one substance selected from the group consisting of OCIF, analogues thereof and variants thereof, which is bound to at least one substance selected from the group consisting of polysaccharides and derivatives thereof, can be distinguished from the free, uncomplexed OCIF or analogue or variant thereof per se using various measures including isoelectric point, sugar content and immunological detection.

The isoelectric point can be measured using any conventional isoelectric electrophoresis technique well-known to the skilled person in the art. OCIF is a basic protein and the isoelectric point thereof is about pI 9. This is significantly higher than that of the complexes of the present invention comprising OCIF and polysaccharides and variants thereof such as dextran sulfate, typical pI values of which are in the region of 5 to 7. Therefore, it is possible to readily distinguish complexed and uncomplexed OCIF using this technique.

The sugar content of the complexes of the present invention and of free, uncomplexed OCIF or an analogue or variant thereof can be measured using any technique conventionally used to quantify neutral sugar content, typical examples including the phenol sulfuric acid method [M. Dubois et al., Anal. Chem., **28**, 350 (1956)]. Since the total sugar content of a complex of the present invention comprising OCIF or an analogue or variant thereof and a polysaccharide or a variant thereof is greater than that of OCIF itself, they can be distinguished from each other.

A further alternative method for distinguishing free, uncomplexed OCIF or an analogue or variant thereof from the complexes of the present invention comprising said OCIF or an analogue or variant thereof bound to a polysaccharide or a variant thereof is to quantify the amount of polysaccharide or variant thereof in each using an antibody which specifically binds to said polysaccharide or variant.

In order to measure the amount of protein in an OCIF or an analogue or variant thereof or in a complex of the present invention comprising OCIF or an analogue or variant thereof and a polysaccharide or variant thereof, any technique conventionally used to measure total protein content can be used. Suitable examples include Lowry's method [Lowry, O.H. *et* al, J. Biol. Chem, **193**, 265-275 (1951)], absorbance ($\lambda$ 280 nm) silver staining and the BCA method.

Free, uncomplexed OCIF or an analogue or variant thereof, or OCIF or an analogue or variant thereof present in a complex of the present invention can be measured immunologically using a method that employs at least one anti-OCIF monoclonal antibody. Examples of a suitable anti-OCIF monoclonal antibody preferably used for the immunological measurement of human OCIF include an antibody produced by hybridoma OI-19 (FERM BP-6420), an antibody produced by hybridoma OI-4 (FERM BP-6419) and an antibody produced by hybridoma OI-26 (FERM BP-6421) (e.g. see WO-A-99/15691). These antibodies are referred to as "antibody OI-19", "antibody OI-4", and "antibody OI-26", respectively, in the present invention. The antibody OI-19 and antibody OI-4 bind both OCIF monomer and OCIF dimer at an equivalent affinity, while antibody OI-26 specifically binds the OCIF dimer. Immunological measurement can be performed using antibodies of this type according to any method well-known to the person skilled in the art (e. g. see WO-A-99/15691). Examples of suitable methods include enzyme immunoassay (referred to as "EIA"), radio immunoassay, enzyme-linked immunosorbent assay (ELISA) and sandwich EIA. Of these, ELISA is preferred. Where the OCIF is of human origin, ELISA can preferably be employed using antibody OI-19 or antibody OI-26 as the immobilized antibody and antibody OI-4 as the enzyme-labeled antibody. The preferred enzyme used for labeling the antibody is peroxidase (referred to as "POD").

Hybridoma producing antibody OI-4 was deposited domestically as "OI-4" at the National Institute of Bioscience and Human-Technology Agency of Industrial Science and Technology at 1-3, Higashi 1 chome, Tsukuba-shi Ibaraki-ken 305-8566 Japan (which has since become the International Patent Organism Depositary, National Institute of Advanced Industrial Science and Technology at AIST Tsukuba Central 6, 1-1, Higashi 1-Chome Tsukuba-shi, Ibaraki-ken 305-8566 Japan) on 16 October 1997 (Heisei-9) and a deposition number FERM P-16473 was granted. It was transferred to an international deposition with the deposition number FERM BP-6419 on 13 July 1998 (Heisei-10).

Hybridoma producing antibody OI-19 was deposited domestically as "OI-19" at the National Institute of Bioscience and Human-Technology Agency of Industrial Science and Technology at 1-3, Higashi 1 chome, Tsukuba-shi Ibaraki-ken 305-8566 Japan (which has since become the International Patent Organism Depositary, National Institute of Advanced Industrial Science and Technology at AIST Tsukuba Central 6, 1-1, Higashi 1-Chome Tsukuba-shi, Ibaraki-ken 305-8566 Japan) on 16 October 1997 (Heisei-9) and a deposition number FERM BP-16474 was granted. It was transferred to an international deposition with a deposition number FERM BP-6420 on 13 July 1998 (Heisei-10).

Hybridoma producing antibody OI-26 was deposited domestically as "OI-26" to National Institute of Bioscience and Human-Technology Agency of Industrial Science and Technology at 1-3, Higashi 1 chome, Tsukuba-shi Ibaraki-ken 305-8566 Japan (which has since become the International Patent Organism Depositary, National Institute of Advanced Industrial Science and Technology at AIST Tsukuba Central 6, 1-1, Higashi 1-Chome Tsukuba-shi, Ibaraki-ken 305-8566 Japan) on 16 October 1997 (Heisei-9) and a deposition number FERM P-16475 was granted. It was transferred to an international deposition with a deposition number FERM BP-6421 on 13 July 1998 (Heisei-10) (see WO-A-99/15691).

The blood or serum concentration of a complex of the present invention comprising OCIF or an analogue or variant thereof and a polysaccharide or a variant thereof can be measured as follows. First, said complex is administered to a human or non-human animal. Then, after a defined length of time, blood or serum is recovered therefrom. The blood or serum concentration of said complex is then measured by ELISA using at least one anti-OCIF monoclonal antibody as described elsewhere in the present application (see WO-A-99/15691).

The complex of the present invention comprising at least one substance selected from the group consisting of OCIF, analogues thereof and variants thereof, which is bound to at least one substance selected from the group consisting of polysaccharides and derivatives thereof is useful for treating or preventing bone metabolic diseases. In the present invention, bone metabolic diseases are any diseases which are characterized by a decreased net amount of bone in the patient suffering therefrom and in which it is necessary to suppress bone resorption and/or the rate of bone resorption in order to treat or prevent said diseases. Bone metabolic diseases that can be treated or prevented by the complex of the present invention include: primary osteoporosis (senile osteoporosis, postmenopausal osteoporosis and idiopathic juvenile osteoporosis); endocrine osteoporosis (hyperthyroidism, hyperparathyroidism, Cushing's syndrome and acromegaly); osteoporosis accompanying hypogonadism (hypopituitarism, Klinefelter syndrome and Turner syndrome); hereditary and congenital osteoporosis (osteogenesis imperfecta, homocystinuria, Menkes syndrome, and Riley-Day syndrome); osteopenia due to gravity load mitigation or fixation and immobilization of limbs; Paget's disease; osteomyelitis; infectious focus due to loss of bone; hypercalcemia resulting from solid carcinoma (e. g. breast carcinoma, lung cancer, kidney cancer and prostatic cancer); a hemology-malignant disease (multiple myeloma, lymphoma and leukemia); idiopathic hypercalcemia; hypercalcemia accompanying hyperthyroidism or kidney malfunction; osteopenia resulting from steroid medication; osteopenia resulting from administration of other medicines (e.g. immunosuppresants such as methotrexate and cyclosporin A, heparin and antiepileptics); osteopenia resulting from kidney malfunction; osteopenia resulting from a surgical operation or digestive organ disease (e.g. small intestine hindrance, large intestine hindrance, chronic hepatitis, gastrectomy, primary biliary liver cirrhosis and liver cirrhosis); osteopenia due to different types of rheumatism such as rheumatoid arthritis, osteoclasis; joint destruction due to different types of rheumatism such as rheumatoid arthritis; mucilance type rheumatism; osteoarthritis; loss of periodontal bone; cancer metastasis of bone (osteolysis metastasis); osteonecrosis or osteocyte death accompanying traumatic injury, Gaucher's disease, sickle cell anemia, lupus erythematosus systemic or nontraumatic injury; osteodystrophy such as renal osteodystrophy; osteopenia accompanying hypoalkalinephosphatasemia or diabetes; osteopenia accompanying nutritional disorders or eating disorders; and other osteopenia. Bone metabolic diseases also include cachexia due to solid carcinoma or cancer metastasis of bone or hemology-malignant disease (see Japanese patent application Publication 2000-178200).

A composition which comprises a complex of the present invention comprising at least one substance selected from the group consisting of OCIF, analogues thereof and variants thereof which is bound to at least one substance selected from the group consisting of polysaccharides and derivatives thereof together with a pharmaceutically acceptable carrier or diluent therefore can be safely administered orally or non-orally to a human or non-human animal. The dosage form can be suitably selected and will vary depending on various factors such as the type of disease being treated, the extent of said disease, and the age, sex and weight of the patient. For example, the complex may be administered orally in the form of tablets, capsules, powders, granules or syrups, injected intravenously alone or in combination with conventional adjuncts such as glucose, amino acids or the like, injected intramuscularly, subcutaneously, intracutaneously or intraperitoneally alone, administered transdermally in the form of cataplasma, administrated transnasally in the form of a nasal drop, administered transmucosaly or to the oral cavity in the form of a mucous membrane applying agent, or administered intrarectally in the form of suppository. These preparations can be formulated in a conventional manner using well-known additives generally used in the field of medicine, such as excipients, binding agents, disintegrants, lubricants, flavoring agents, solubilizers, suspending agents, colorants, pH regulators, antiseptics, gelling agents, surfactants and coating agents.

Where the complexes of the present invention are formulated as tablets, any carriers known in the art can be used. The carriers include, for example, excipients such as lactose, white sugar, sodium chloride, glucose, urine, starch, calcium carbonate, kaolin, crystalline cellulose, silicate or the like; binding agents such as water, ethanol, propanol, simple syrup, glucose solution, starch solution, gelatin solution, carboxymethyl cellulose, shellac, methyl cellulose, potassium phosphate, polyvinyl pyrrolidone or the like; disintegrants such as dry starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch, lactose or the like; decomposition inhibitors such as white sugar, stearin, cacao butter, hydrogenated oil or the like; absorption accelerators such as quaternary ammonium bases, sodium lauryl sulfate or the like; moisturizers such as glycerin, starch or the like; adsorbents such as starch, lactose, kaolin, bentonite, colloidal silicate or the like; and lubricants such as refined talc, stearic acid, metal salts of stearic acid such as calcium stearate and magnesium stearate, talc, boric acid powder, polyethylene glycol or the like. In addition, if desired the tablets may be coated, for example, to form a sugar coated tablet, a gelatin coated tablet, an enteric coated tablet, a film coated tablet, a two-layered tablet or a multilayered tablet.

Where the complexes of the present invention are formulated as pilules, the preparation may contain carriers known in the art, for example, excipients such as glucose, lactose, cacao butter, starch powder, hardened vegetable oil, kaolin, talc or the like; binding agents such as gum arabic powder, tragacanth powder, gelatin, ethanol or the like; and disintegrants such as laminaran, agar or the like.

Where the complexes of the present invention are formulated as a suppository, the preparation may contain conventional carriers such as polyethylene glycol, cacao butter, higher alcohols, esters of higher alcohols, gelatin, semi-synthesized glyceride or the like.

Where the complexes of the present invention are formulated as injections, it is preferable that the preparation in the form of a solution or suspension is sterilised and is made isotonic with blood. When the preparations are in the form of a solution, emulsion or suspension, any diluent known and conventionally used in the art can be employed, examples of which include water, ethanol, propylene glycol, ethoxylated isostearyl alcohol, polyoxylated isostearyl alcohol and polyoxyethylene sorbitan fatty acid esters. Additionally, in such injectable formulations, the preparations may also contain salts, glucose, glycerin or the like in an amount sufficient to maintain isotonicity with blood. They may also contain further agents including solubilizers, buffering agents, soothing agents, pH regulators, stabilizers and solubilizing agents. The injections can be freeze-dried after formulation.

The preparations of the present invention may also contain further additives such as coloring agents, preservatives, perfumes, flavoring agents, sweeteners or other medicines.

There is no specific limitation on the amount of the complex of the present invention comprising at least one substance selected from the group consisting of OCIF, analogues thereof and variants thereof and at least one substance selected from the group consisting of polysaccharides and variants thereof that is present in the composition for administration in order to prevent or treat bone metabolic disease, but it is usually 0.1 to 70 % by weight, and preferably it is 1 to 30 % by weight of the whole formulation.

The dose of the complex according to the present invention will vary depending on a variety of factors including the condition to be treated, the age, sex and body weight of the patient and the administration route. However, the amount administered to an adult human is generally in a range having an upper limit of from 30 to 1000 mg and a lower limit of from 0.001 to 0.03 mg per day. The preferred range is from 0.03 to 30 mg per day. The amount administered is generally in a range having an upper limit of from 1 to 20 mg/kg per day and a lower limit of from 0.01 to 0.5 μg/kg per day. The preferred range is from 0.5 μg/kg to 1 mg/kg per day. The complex of the invention can be administered once per day or more than once per day, depending on factors such as the form of administration and the condition of the patient.

The following examples, reference examples and test examples are intended to further illustrate the present invention and are not intended to limit the scope of this invention in any way.

Example 1

Preparation of Complexes Comprising OCIF and Dextran Sulfate (I)

1(a) Preparation of Recombinant Dimeric Human OCIF

Recombinant dimeric human OCIF having a molecular weight of about 120000 was obtained according to the procedure described in examples of EP-A-0816380 (WO-A-96/26217). Namely, pBKOCIF, a plasmid vector comprising a nucleotide sequence that encodes human OCIF containing a signal peptide, obtained from the *E. coli* transformant strain pBK/01F10 [deposited as FERM BP-5267 under the Budapest Treaty at the National Institute of Bioscience and Human-Technology, Agency of Industrial Science and Technology at 1-3, Higashi 1 chome, Tsukuba-shi Ibaraki-ken 305-8566 Japan (which has since become the International Patent Organism Depositary, National Institute of Advanced Industrial Science and Technology)] produced according to Example 11 of EP-A-0816380, was digested with restriction enzymes Sal1 and EcoRV. The nucleotide that encodes human OCIF containing a signal peptide, which is equivalent to human OCIF cDNA, was recovered according to the procedure described in Example 14 of EP-A-0816380. After separation and purification of said nucleotide, it was inserted into the expression vector pcDL-SR $\alpha$296 (Molecular and Cellular Biology, vol 8, p466, 1988), and then *E. coli* strain DH5 $\alpha$ (Gibco BRL), was transformed thereby (see the procedure described in Example 14 of EP-A-0816380). The recombinant vector named pSR$\alpha$OCIF thus obtained was extracted from said transformant culture and purified.

The procedure of Example 14 of EP-A-0816380 was then applied to obtain the desired recombinant human mature OCIF. Namely, CHO dhFr- cells (ATCC, CRL 9096) were transfected with the recombinant plasmid pSR$\alpha$OCIF produced above and a plasmid expressing dihydrofolate reductase (DHFR) (plasmid pBAdDSV disclosed in WO-A-92/01053) and then a DHFR-expressing transfectant was selected. The transformants that expressed large amounts of OCIF were cloned. The clones whose conditioned medium contained OCIF at a high concentration were selected and the clone expressing the largest amount of OCIF, 5561, was obtained. A culture of clone 5561 thus obtained was conditioned and filtrated, and then applied to a Heparin Sepharose-FF column (2.6 x 10 cm, Pharmacia Co.) and subjected to column chromatography using a linear sodium chloride gradient as the eluant. The fraction having OCIF activity eluted with approximately 0.6 to 1.2 M sodium chloride was then applied to an affinity column (blue-5PW, 0.5 x 5.0 cm, Tosoh Co) and subjected to affinity chromatography using a linear sodium chloride gradient as the eluant. The eluted fractions were subjected to SDS-polyacrylamide gel electrophoresis under reducing and non-reducing conditions and the fractions containing the desired purified recombinant human mature OCIF were designated to be those that produced the same bands of rOCIF protein with apparent molecular weights of 60000 and 120000 as produced in Example 14 of EP-A-0816380. The amino acid sequence of the monomeric peptide is shown in SEQ. ID. NO.1 of the sequence listing, which is identical with the full sequence of SEQ. ID. NO.4 or the amino acids No.1 to No.380 of SEQ. ID. NO.5 of WO-A-96/26217 and EP-A-0816380.

The combined fractions containing the obtained human OCIF was then supplemented with 1/100 volume of 25% trifluoroacetic acid and the resulting mixture was applied to a reverse phase column (PROTEIN-RP, 2.0 mm x 250 mm, purchased from YMC Co.) that had been pre-equilibrated with 30% acetonitrile containing 0.1% trifluoroacetic acid. The column was then eluted with a linear gradient of from 30% to 55% acetonitrile at a flow rate of 0.2 ml/min for 50 min. Two peak fractions were collected separately and then lyophilized. The fraction which showed a band having an apparent molecular weight of 120000 on SDS-PAGE under reducing conditions was then employed in the following examples as the dimeric human OCIF (see Examples 17 and 18 of WO-A-96/26217 and EP-A-0816380).

1(b) Preparation of Complexes Comprising OCIF and dextran sulfate

Purified dimeric human OCIF, prepared as described in Example 1(a) above, was dissolved in 10 mM sodium phosphate buffer solution (pH 6.0) containing 0.15 M sodium chloride to give solutions with an OCIF concentration of 1.5, 2, 5, 6.5, 10, 12.5, 20 or 50 mg/ml. Dextran sulfate sodium salt sulfur 5 (manufactured by Meito Sangyo Co., Ltd., hereinafter referred to as "DS5") was dissolved in the aqueous solutions thus produced to a final concentration of 40, 100, 130, 150, 200, 400, 500, 510 or 1000 mg/ml, and then 1 N sodium hydroxide was added thereto to a final pH of 10, 10.5 or 11. The obtained aqueous solutions were incubated at 4, 7, 25 or 37 °C for 1, 3, 6, 18, 24, 48, 72, 96, 144, 168 or 288 hours.

At the end of this time, 4 ml of each resulting solution were applied to a Superdex 200 prep grade gel filtration column (inside diameter of the column: 16 mm; length: 60 cm, exclusion-limiting molecular weight: 1,300,000; manufactured by Amersham Pharmacia Biotech) previously equilibrated with 10 mM sodium phosphate buffer (pH 6) containing 0.3 M sodium chloride, and then eluted with the same buffer at a flow rate of 2 ml/min. Absorption at wavelength 280 nm was monitored using an ultraviolet spectrophotometer, and the eluate at a retention time of about 28 to 36 minutes was collected. Free DS5 which had not bound to the OCIF was eluted at a retention time of about 50 to 70 minutes. All steps of this gel filtration procedure were performed at room temperature. The obtained preparations which contained the desired complexes of dimeric human OCIF and DS5 were frozen and stored at -60 °C. The preparation conditions for each complex are summarized in Table 1 below.

Table 1

| Prep. number | DS5 conc. (mg/ml) | OCIF conc. (mg/ml) | Temp. (°C) | pH | Incubation Time (hours) |
|---|---|---|---|---|---|
| Prep. 1 | 130 | 6.5 | 4 | 10.5 | 18 |
| Prep. 2 | 510 | 6.5 | 4 | 10.5 | 18 |
| Prep.3 | 130 | 6.5 | 4 | 11 | 18 |
| Prep. 4 | 130 | 6.5 | 4 | 10.5 | 72 |
| Prep. 5 | 500 | 5 | 4 | 10.5 | 144 |
| Prep. 6 | 130 | 6.5 | 4 | 10.5 | 48 |
| Prep. 7 | 130 | 6.5 | 4 | 10.5 | 144 |
| Prep. 8 | 130 | 6.5 | 4 | 10.5 | 288 |
| Prep. 9 | 400 | 20 | 4 | 10.5 | 18 |
| Prep. 10 | 200 | 10 | 4 | 10.5 | 18 |
| Prep. 11 | 100 | 5 | 4 | 10.5 | 18 |
| Prep. 12 | 40 | 2 | 4 | 10.5 | 18 |
| Prep. 13 | 1000 | 12.5 | 4 | 10.5 | 18 |
| Prep. 14 | 1000 | 50 | 4 | 10.5 | 18 |
| Prep. 15 | 400 | 2 | 4 | 10.5 | 144 |
| Prep. 16 | 1000 | 5 | 4 | 10.5 | 18 |
| Prep. 17 | 1000 | 2 | 4 | 10.5 | 18 |
| Prep. 18 | 150 | 5 | 37 | 10.5 | 1 |
| Prep. 19 | 150 | 5 | 37 | 10.5 | 3 |
| Prep.20 | 150 | 5 | 37 | 10.5 | 6 |
| Prep.21 | 150 | 5 | 37 | 10.5 | 24 |
| Prep.22 | 150 | 5 | 7 | 10.5 | 168 |
| Prep.23 | 150 | 5 | 4 | 10 | 144 |
| Prep.24 | 150 | 5 | 25 | 10 | 24 |
| Prep.25 | 130 | 6.5 | 4 | 10.5 | 24 |
| Prep.26 | 150 | 5 | 37 | 10 | 24 |
| Prep.27 | 150 | 5 | 4 | 10.5 | 144 |
| Prep.28 | 150 | 5 | 4 | 11 | 24 |
| Prep.29 | 150 | 5 | 4 | 10.5 | 24 |
| Prep. 30 | 150 | 1.5 | 4 | 10.5 | 72 |
| Prep. 31 | 130 | 6.5 | 25 | 10.5 | 1 |
| Prep. 32 | 130 | 6.5 | 25 | 10.5 | 3 |

Table 1   (continued)

| Prep. number | DS5 conc. (mg/ml) | OCIF conc. (mg/ml) | Temp. (°C) | pH | Incubation Time (hours) |
|---|---|---|---|---|---|
| Prep. 33 | 130 | 6.5 | 25 | 10.5 | 6 |
| Prep.34 | 130 | 6.5 | 25 | 10.5 | 24 |
| Prep. 35 | 130 | 6.5 | 25 | 10.5 | 168 |
| Prep. 36 | 130 | 6.5 | 25 | 10.5 | 288 |
| Prep. 37 | 150 | 5 | 4 | 10.5 | 96 |
| Prep. 38 | 150 | 5 | 4 | 10.5 | 288 |
| Prep. 39 | 130 | 6.5 | 25 | 10.5 | 18 |
| Prep. 40 | 130 | 6.5 | 37 | 10.5 | 18 |

1(c) Preparation of Natural Human OCIF

Naturally-produced human OCIF was prepared according to the procedure described in Examples 1 to 4 of WO-A-96/26217 and EP-A-0816380 from a culture of human fetal lung fibroblast cell IMR-90 (ATCC-CCL186).

Example 2

Preparation of Complexes Comprising OCIF and Dextran Sulfate (II)

Purified dimeric human OCIF, prepared as described in Example 1(a) above, was dissolved in 10 mM sodium phosphate buffer solution (pH 6.0) containing 0.15 M sodium chloride to give a solution having an OCIF concentration of 5 mg/ml. Dextran sulfate sodium salt having a molecular weight of 5000 (manufactured by Wako Pure Chemical Industries, Ltd., hereinafter referred to as "DS 5000") was dissolved in the aqueous solution thus obtained to give a final concentration of DS 5000 of 150 mg/ml, and then 1 N sodium hydroxide was added thereto to a final pH of 10.5. The aqueous solution thus obtained was incubated at 4 °C for 24 hours.

At the end of this time, 4 ml of the resulting solution were applied to a Superdex 200 prep grade gel filtration column chromatography as described in Example 1(b) above. Absorption at wavelength 280 nm was monitored using an ultraviolet spectrophotometer, and the eluate at a retention time of about 28 to 36 minutes was collected. Free DS 5000 which had not bound to the OCIF was eluted at a retention time of about 40 to 65 minutes.

The obtained preparations which contained the desired complexes of dimeric human OCIF and DS5000 were frozen and stored at -60 °C. The preparation conditions for the complex are summarized in Table 2 below.

Table 2

| Prep. number | DS5000 Conc. (mg/ml) | OCIF Conc. (mg/ml) | Temp. (°C) | pH | Incubation time (hours) |
|---|---|---|---|---|---|
| Prep.41 | 150 | 5 | 4 | 10.5 | 24 |

Example 3

Measurement of Isoelectric Point

The purified recombinant dimeric human OCIF, prepared as described in Example 1(a) above and the complex of OCIF and dextran sulfate prepared in Example 1(b) above and which is designated Preparation Number 22 in Table 1 were applied separately to an isoelectric electrophoresis gel IEF PAGE mini (pH range of 3 to 10, manufactured by Iwaki Glass), using an IEF pH 3-7 buffer kit (Technical Frontier Co.) and a voltage was applied to the gel according to the following regime: 100 V for 1 hour, followed by 200 V for 1 hour and finally 500 V for 30 minutes. After completion of the electrophoresis, the resultant gel obtained in each case was stained with Coomassie Blue.

From the electrophoresis gels obtained above, it was determined that the isoelectric point of the dimeric human OCIF was about pI 9, and the isoelectric point of the complex of OCIF and dextran sulfate designated Preparation Number 22 was about pI 6.5 by comparing the band position of OCIF and that of the OCIF complex with pI markers.

Example 4

Measurement of the Molecular Ratio of OCIF and Dextran Sulfate in a Complex Comprising OCIF and Dextran Sulfate

4(a) Preparation of a Stock Solution of an Anti-Human OCIF Monoclonal Antibody OI-4 Labeled with Peroxidase

In this step, anti-human OCIF monoclonal antibody was labeled with peroxidase using an EZ-Link Maleimide Activated Horseradish Peroxidase Kit (manufactured by Pierce) according to the protocol II described in the instruction booklet supplied with the kit. Details of this procedure are as follows.

Anti-human OCIF monoclonal antibody OI-4 was purified from a culture of a hybridoma producing antibody OI-4 (FERM BP-6419) according to the method described in Example 4 of EP-A-0974671 (WO-A-99/15691), and then diluted to a final protein concentration of 1mg/ml with 10mM phosphate buffer (pH 7.6).

N-succinimidyl S-acetylthioacetate (provided in said EZ-Link Maleimide Activated Horseradish Peroxidase Kit) was dissolved in dimethylformamide to give a solution having a concentration of 10 mg/ml just before use. A 4µl aliquot thereof was added to 1 ml of the diluted OI-4-containing solution prepared above, and the resulting solution was then incubated at room temperature for 30 minutes. At the end of this time, 20 µl of a solution obtained just before it was needed by dissolving 5 mg of hydroxylamine hydrochloride in 100 µl of Maleimide Conjugation Buffer (provided in said EZ-Link Maleimide Activated Horseradish Peroxidase Kit) were added thereto, and the resulting solution was incubated at a room temperature for 2 hours. At the end of this time, the reaction mixture was applied to a polyacrylamide desalting column (10 ml, contained in said EZ-Link Maleimide Activated Horseradish Peroxidase Kit) previously equilibrated with 30 ml of Maleimide Conjugation Buffer (also provided in said kit), and then Maleimide Conjugation Buffer was applied to said column. The eluate was collected in 0.5 ml fractions. The 7th to 10th fractions containing the antibody were combined. 100 µl of a solution obtained by dissolving 5 mg of EZ-Link Maleimide Activated Horseradish Peroxidase (contained in said EZ-Link Maleimide Activated Horseradish Peroxidase Kit) in 500 µl of distilled water just before it was needed were then added to the combined eluate fractions and the resulting mixture was incubated at room temperature for one hour. After incubation, an equal volume of glycerol was added thereto, and the solution thus obtained was stored at -20 °C.

The solution obtained by the above process was used as a stock solution of the anti-human OCIF monoclonal antibody OI-4 labeled with peroxidase (hereinafter referred to as "POD-OI-4"), and is referred to hereinafter as "POD-OI-4 stock solution".

4(b) Quantification of OCIF

The amount of OCIF present in any of the complexes prepared in Examples 1 and 2 above and the combination prepared in Reference Example 1 below was measured by enzyme-linked immunosorbent assay (ELISA) using two anti-OCIF monoclonal antibodies, the details of the procedure being as follows.

Anti-human OCIF monoclonal antibody OI-26 was purified from a culture of a hybridoma producing antibody OI-26 (FERM BP-6421) according to the method described in Example 4 of EP-A-0974671 (WO-A-99/15691), and then dissolved in 0.1 M sodium hydrogen carbonate to give a solution having a final protein concentration of 5 µ g/ml. A 100 µl aliquot thereof was transferred to each well of a 96-well microtitre plate (Maxisorp, manufactured by NUNC), and the plate was then sealed and incubated at 4 °C overnight. At the end of this time, each well was washed three times with 250 µl of phosphate buffered saline (PBS) (pH 7.4) containing 0.1 % Polysorbate 20. 20 µl of a dilution buffer solution [comprising 0.2 M Tris-hydrochloric acid, 40 % Block Ace (purchased from Dainippon Pharmaceutical Co., Ltd.), and 0.1% Polysorbate 20; pH 7.4] were added to each well, and then the plate was kept at room temperature for 20 minutes to block areas of the well unbound by OI-26.

The samples to be added to the OI-26 bound wells prepared above were preferably diluted with the dilution buffer solution used above to block the wells. In order to make a calibration curve, the dilution buffer solution containing human OCIF at known concentrations was used as standards. The dilution buffer solution was used as a control. 50 µl of each sample were transferred to each well.

After addition of the samples to the wells, 50 µl of a solution obtained by diluting the POD-OI-4 stock solution [prepared as described in Example 4(a) above] 1500-fold volume with a dilution buffer solution [0.2 M Tris-hydrochloric acid, 40 % Block Ace (purchased from Dainippon Pharmaceutical Co., Ltd.), 0.1 % polysorbate 20 (pH 7.4)] were added to each well and the plate was then incubated at room temperature for 2 hours. At the end of this time, each well was washed four times with 250 µl of phosphate buffer containing 0.1 % polysorbate 20 (hereinafter referred to as "PB", pH 7.4).

0.1 M citric acid and 0.2 M disodium hydrogenphosphate were mixed, and used as a substrate solution (pH 4.5). A 32.5 ml aliquot thereof was transferred to a test tube and 6.5 µl of hydrogen peroxide were added thereto. 13 mg of an o-phenylenediamine dihydrochloride (OPD) tablet (manufactured by Wako Pure Chemical Industries, Ltd.)

were then dissolved in the resulting solution. A 100 µl aliquot thereof was added to each well, the plate was covered with aluminum foil, and then it was incubated at room temperature for 15 minutes. At the end of this time, 50 µl of a reaction stopping solution comprising purified water and concentrated sulfuric acid in a ratio of 250:50 by volume were added to each well. After stirring the solutions in the wells gently with a shaker (Titer mixer MB-1: manufactured by Japan Trika), the absorbance of each well at a wavelength of 490 nm was measured by a microplate reader (SPECTRA FLUOR: manufactured by TECAN).

On the basis of the calibration curve produced as explained above from the abosorbance of standard solutions of human OCIF at known concentrations, the amount of human OCIF in each sample was calculated.

4(c) Quantification of Dextran Sulfate

The amount of dextran sulfate in each complex produced as described in Examples 1 and 2 above was measured as a neutral sugar by the phenol sulfuric acid method, the details of which are as follows.

A solution having a known concentration in the range of 10 to 60 µg/ml of DS5 (manufactured by Meito Sangyo Co., Ltd.) or DS5000 (manufactured by Wako Pure Chemical Industries, Ltd.) was prepared using a diluting solution (0.01 M citric acid, 0.3 M sodium chloride, 0.01% polysorbate 80 aqueous solution: pH 6.0), and used as a standard solution. 0.2 ml each of the standard, a sample, or the diluting solution were transferred to each test tube. 0.2 ml of 50 mg/ml aqueous phenol were added thereto, and stirred rapidly. After incubating the resulting mixture at 60 °C for 20 seconds in a water bath, 1.0 ml of concentrated sulfuric acid was added thereto. After gentle but rapid stirring, the tube was incubated for 10 minutes at room temperature, stirred rapidly again, and then incubated for 20 minutes at room temperature. At the end of this time, the absorbance of the solution in the tube at a wavelength of 490 nm was measured using a spectrophotometer (UV-240: manufactured by Shimadzu Seisakusho, K. K.).

From this absorbance and a calibration curve, the neutral sugar content was determined. Human OCIF contains a sugar chain. Therefore, the amount of dextran sulfate bound to human OCIF in the preparation being analysed was calculated by deducting the value of the neutral sugar content of human OCIF itself from that measured for any preparation being analysed.

4(d) Calculation of the Molecular Ratio of OCIF and Dextran Sulfate in a Complex Comprising OCIF and Dextran Sulfate

The amount of dextran sulfate present in the preparation being analysed, determined as described in Example 4(c) above was divided by the amount of human OCIF present in the preparation being analysed, determined as described in Example 4(b) above to give the amount of dextran sulfate present per 1 mg of human OCIF in the preparation being analysed.

The figure thus obtained was then used to calculate the molecular ratio of OCIF as monomer and dextran sulfate in the preparation being analysed by calculating the number of dextran sulfate molecules per one molecule of OCIF monomer, based on the assumption that the molecular weight of human OCIF monomer is 60000, the molecular weight of DS5 is 1950, the molecular weight of DS5000 is 5000.

The results obtained are shown in the following Table 3

Table 3

| Complex | Amount of dextran sulfate in the complex (µg/mg OCIF) | Molecular ratio of OCIF as monomer and dextran sulfate in complex |
|---|---|---|
| Prep. 1 | 48.7 | 1:1.5 |
| Prep. 2 | 100.2 | 1:3.1 |
| Prep. 3 | 39.7 | 1:1.2 |
| Prep. 4 | 62.0 | 1:1.9 |
| Prep. 5 | 136.4 | 1:4.3 |
| Prep. 6 | 60.7 | 1:1.9 |
| Prep. 7 | 58.5 | 1:1.8 |
| Prep. 8 | 60.3 | 1:1.9 |
| Prep. 9 | 67.7 | 1:2.1 |
| Prep. 10 | 94.3 | 1:2.9 |

Table 3   (continued)

| Complex | Amount of dextran sulfate in the complex (µg/mg OCIF) | Molecular ratio of OCIF as monomer and dextran sulfate in complex |
|---|---|---|
| Prep. 11 | 63.6 | 1:2.0 |
| Prep. 12 | 60.8 | 1:1.9 |
| Prep. 13 | 144.9 | 1:4.5 |
| Prep. 14 | 116.4 | 1:3.6 |
| Prep. 15 | 126.9 | 1:4.0 |
| Prep. 16 | 145.0 | 1:4.5 |
| Prep. 17 | 116.5 | 1:3.6 |
| Prep. 18 | 46.0 | 1:1.4 |
| Prep. 19 | 61.0 | 1:1.9 |
| Prep. 20 | 68.3 | 1:2.1 |
| Prep. 21 | 110.7 | 1:3.4 |
| Prep. 22 | 100.3 | 1:3.1 |
| Prep. 23 | 65.8 | 1:2.1 |
| Prep. 24 | 58.2 | 1:1.8 |
| Prep. 25 | 43.8 | 1:1.4 |
| Prep. 26 | 80.1 | 1:2.5 |
| Prep. 27 | 61.8 | 1:2.0 |
| Prep. 28 | 57.1 | 1:1.8 |
| Prep. 29 | 69.3 | 1:2.2 |
| Prep. 30 | 77.1 | 1:2.4 |
| Prep. 31 | 34.5 | 1:1.1 |
| Prep. 32 | 53.0 | 1:1.7 |
| Prep. 33 | 47.4 | 1:1.5 |
| Prep. 34 | 62.2 | 1:2.0 |
| Prep. 35 | 96.2 | 1:3.0 |
| Prep. 36 | 122.5 | 1:3.9 |
| Prep. 37 | 67.8 | 1:2.1 |
| Prep. 38 | 69.5 | 1:2.4 |
| Prep. 39 | 78.0 | 1:2.5 |
| Prep. 40 | 98.4 | 1:3.1 |
| Prep. 41 | 161.2 | 1:1.9 |

Example 5

The Stability of Binding Between OCIF and Dextran Sulfate in OCIF/Dextran Sulfate Complexes

The gel filtration of a complex comprising OCIF and dextran sulfate was repeated twice as described in Example 4(c) above, and the amount of dextran sulfate present in the complex obtained after each of said gel filtrations was measured. The details are as follows.

5(a) Incubation of OCIF and Dextran Sulfate

The procedure described above in Example 1(b) was used. Recombinant dimeric human OCIF, prepared as described in Example 1(a) above, was dissolved in 10 mM sodium phosphate buffer (pH 6.0) containing 0.15 M sodium chloride to give a solution having an OCIF concentration of 5 mg/ml. DS5 was dissolved in the solution thus obtained to give a final DS5 concentration of 150 mg/ml, and then 1 N sodium hydroxide solution was added thereto to adjust the pH to 10.5. The resulting solution was then incubated at 4 °C for 7 days to give a solution containing a complex of human dimeric OCIF and DS5.

5(b) First Gel Filtration

The solution containing a complex of human dimeric OCIF and DS5 obtained at the end of the incubation in Example 5(a) above was subjected to gel filtration according to the method described in Example 1(b) above. The fractions at a retention time of about 28 to 36 minutes were collected, while free dextran sulfate which was not bound to OCIF was eluted at a retention time of about 50 to 70 minutes.

5(c) Measurement of Protein Content

The amount of protein present in the complex was measured according to Lowry's method [Lowry, O.H. *et al*, J. Biol. Chem, **193,** 265-275 (1951)] as follows.
0.2 g of copper (II) sulfate pentahydrate (Wako Pure Chemical) were dissolved in water to a final volume of 50 ml. 0.4 g of sodium tartrate dihydrate (Wako Pure Chemical) were dissolved in water to a final volume of 50 ml. 20 g of sodium carbonate were dissolved in water to a final volume of 100 ml. The three aqueous solutions thus obtained were mixed in a ratio of 1:1:2 by volume just before use (the resulting solution was referred to as the "A solution"). 10 g of sodium dodecyl sulfate (Nacalai Tesque Inc.) were dissolved in water to a final volume of 200 ml (the resulting solution was referred to as the "B solution"). 3.2 g of sodium hydroxide (Wako Pure Chemical) were dissolved in water to a final volume of 100 ml (the resulting solution was referred to as the "C solution"). A solution, B solution and C solution were mixed at a ratio of 1:2:1 by volume just before use.
Separately, folin-ciocalteu reagent (Wako Pure Chemical) and water were mixed in a ratio of 1:5 by volume just before use. 2.76 g of citric acid, trisodium salt dihydrate (Wako Pure Chemical), 0.13 g of citric acid monohydrate (Wako Pure Chemical), 17.5g of sodium chloride and 0.1 g of polysorbate 80 were dissolved in water to a final volume of 1 L (pH 6.9) to give a solution referred to as the "diluting solution".
9.5 ml of diluting solution were added to 500 µ L of a standard solution of bovine serum albumin (Pierce Co. Ltd.) containing 2 mg/ml of bovine serum albumin (referred to as "BSA") in 0.9% aqueous sodium chloride containing sodium azide at a concentration of less than 0.1% to give a solution referred to as "100 µ g/ml BSA solution". 3.5 ml, 3 ml, 2.5 ml or 2 ml of diluting solution were added to 1.5 ml, 2 ml, 2.5 ml or 3 ml of 100 µ g/ml BSA solution, respectively to give solutions referred to as "30 µ g/ml BSA solution", "40 µ g/ml BSA solution", "50 µ g/ml BSA solution" and "60 µ g/ml BSA solution" respectively. 3 ml of diluting solution were added to 1.5 ml of 60 µ g/ml BSA to give a solution referred to as "20 µ g/ml BSA solution".
The sample whose protein content was to be determined was diluted with diluting solution to give a solution with a final protein concentration of about 40 µ g protein per 1 ml. 1 ml of 20 µ g/ml BSA solution, 30 µ g/ml BSA solution, 40 µ g/ml BSA solution, 50 µ g/ml BSA solution, 60 µ g/ml BSA solution, the diluted sample or diluting solution (n=3) were transferred to a test tube, and 1 ml of alkaline copper reagent was added thereto, and the resulting solution was mixed and incubated at room temperature for 10 minutes. 0.5 ml of the diluted folin-ciocalteu reagent were then added thereto and the resulting solution was mixed and incubated at room temperature for 30 minutes. At the end of this time, the absorbance of each mixture at a wavelength of 750 nm was measured using a cell made of quartz whose width was 10 mm using an ultraviolet spectrophotometer (Lambda 20: Perkin Elmer Co Ltd.). Then, the amount of protein contained in the sample was calculated on the basis of a calibration curve produced using the absorbances of the standard BSA solutions (as a value reduced to an amount of BSA).

5(d) Quantification of Dextran Sulfate

The amount of dextran sulfate bound to human OCIF in the complex that was obtained after the first gel filtration in Example 5(b) above was measured using the procedure described in Example 4(c) above.

5(e) Second Gel Filtration

The combined collected fractions obtained in Example 5(b) above were transferred to two Centriprep filter units (YM-30, 30,000 MW cutoff, Millipore Amicon Co Ltd.), and they were centrifuged at 2000 rpm for 20 min using a centrifuge machine (himacCT60, Hitachi Seisakusho Co Ltd.). The unfiltered concentrated solutions obtained from the two Centriprep filter units were collected and combined. The resulting solution was subjected to gel filtration as described in Example 1(b) above, and the fractions at a retention time of about 28 to 36 minutes were collected and combined. Then, the protein and sugar content in the complex present in the combined fractions was measured as described in Examples 5(c) and 5(d) above.

5(f) Third Gel Filtration

The combined collected fractions obtained in Example 5(e) above were transferred to two Centriprep filter units (YM-30, 30,000 MW cutoff, Millipore Amicon Co Ltd.), and they were centrifuged at 2000 rpm for 20 min using a centrifuge machine (himacCT60, Hitachi Seisakusho Co Ltd.). The unfiltered concentrated solutions in the two Centriprep filter units were collected and combined. The obtained concentrate was subjected to gel filtration as described in Example 1(b) above, and the fractions at a retention time of about 28 to 36 minutes were collected and combined. Then, the protein and sugar content in the complex present in the combined fractions was measured as described in Examples 5(c) and 5(d) above.

5(g) Calculation of the Molecular Ratio of OCIF to Dextran Sulfate

The molecular ratio of OCIF as monomer to dextran sulfate present in the complex contained in the fractions obtained after the first gel filtration in Example 5(b) above, the second gel filtration in Example 5(e) above and the third gel filtration in Example 5(f) above were calculated according to Example 4(d) above. The results obtained are summarized in Table 4 below.

Table 4

| Gel Filtration | Molecular ratio of OCIF as monomer to dextran sulfate in the complex |
|---|---|
| First | 1:2.2 |
| Second | 1:2.3 |
| Third | 1:2.1 |

It will be immediately apparent from the above that the molecular ratio of OCIF to dextran sulfate in the complex of the present invention is remarkably constant throughout the three gel filtrations, indicating the high degree of stability of the binding between OCIF and dextran sulfate in the complexes of the present invention.

Example 6

The Degree of Adsorption a Complex of OCIF and Dextran Sulfate to a Heparin Cross-Linked Column

6(a) Heparin Column Chromatography

All the column chromatography procedures in this example were performed at a flow rate of 4 ml per minute.
A heparin cross-linked column (HiTrap Heparin HP column, Lot.289212, Amersham Pharmacia Biotech) was pre-equilibrated with 5 ml of 10 mM sodium phosphate buffer containing 0.7 M sodium chloride. A preparation from Table 1 of Example 1 was taken and diluted to a final protein concentration of 0.1 mg/ml with 10 mM sodium phosphate buffer containing 0.7 M sodium chloride. 1 ml of the diluted solution thus obtained was applied to said column and 1 ml of a first eluate was collected (fraction A). Next, 5 ml of 10 mM sodium phosphate buffer containing 0.7 M sodium chloride were applied to said column and 5 ml of a second eluate were collected (fraction B). Finally, 4 ml of 10 mM sodium phosphate buffer containing 2M sodium chloride were applied to said column and 4 ml of an eluate were collected (fraction C).

6(b) Measurement of the Amount of OCIF in the Eluate

100 $\mu$L of 0.1 M sodium hydrogen carbonate (pH 9.6), in which was dissolved an anti-human OCIF monoclonal antibody OI-19 (FERM BP-6420) at a concentration of 10 $\mu$g per ml, were transferred to each well of a 96-well microtitre plate (Maxisorp: NUNC Co Ltd.). The plate was sealed and then incubated at 4 °C overnight. At the end of this time, the solution in each well was removed by decantation, 300 $\mu$L of 50% Block Ace (purchased from Dainippon Pharmaceutical Co., Ltd.) were added to each well, and then the plate was incubated at room temperature for 2 hours. After removing the solution in each well, each well was washed three times with 300 μL of PBS (pH 7.4) containing 0.1% polysorbate 20 using a SERA WASHER MW-96R (Bio Tec Co Ltd.).

After preparing the wells as described above, 20 μL of each of the three eluates (fractions A, B and C) obtained in Example 6(a) above were diluted to a final volume of 120 μL with 0.2 M Tris-HCl (pH 7.4) containing 40% Block Ace, 10 μg/ml of mouse immunoglobulin G and 0.1% polysorbate 20, and then diluted with the same volume of pure water. At the same time, a known amount of human OCIF was dissolved in 120 μL of 0.2 M Tris-HCl (pH 7.4) containing 40% Block Ace, 10 μg/ml of mouse immunoglobulin G and 0.1% polysorbate 20, and then diluted with the same volume of pure water. The solution thus obtained was used as a standard.

100 μL of each of the diluted eluates and of the standard were added to one well each of the pre-prepared microtitre plate described above and then the plate was incubated at room temperature for 2 hours with gentle mixing using a microplate mixer (NS-P: Iuchi Seiei-Do, Co Ltd.). At the end of this time, the solution was removed from each well, and then each well was washed six times with 300 μL of PBS (pH 7.4) containing 0.1% polysorbate 20 using a SERA WASHER MW-96R (Bio Tec Co Ltd.). 100 μL of 0.1 M Tris-HCl (pH 7.4) containing 25% Block Ace, 10 μg/ml of mouse immunoglobulin G and 0.1% polysorbate 20, to which had been added the POD-OI-4 stock solution prepared in Example 4(a) above to give a 0.01% solution (volume per volume), were then added to each well and the plate was incubated at room temperature for 2 hours with gentle mixing using the same microplate mixer. After removing the solution in each well, the well was washed six times with 300 μL of PBS (pH 7.4) containing 0.1% polysorbate 20 using a SERA WASHER MW-96R (Bio Tec Co Ltd.).

After the wells had been washed, 100 μL of 3,3', 5,5'-tetramethylbenzidine (TMB) soluble reagent (Scytek Co Ltd.) were added to each well and the plate was then incubated at room temperature for 10 to 15 minutes with gentle mixing using the same microplate mixer as above. At the end of this time, 100 μL of TMB stop buffer (Scytek Co Ltd.) were added to each well. After mixing the plate gently with the microplate mixer for about 1 minute, the absorbance of each well at a wavelength of 450 nm was measured using a microplate reader (SPECTRA THERMO : TECAN Co Ltd.). The amount of OCIF contained in each of fractions A, B and C [designated (a), (b) and (c)] was then calculated on the basis of a calibration curve prepared by plotting the absorbance of each standard described above against concentration. The degree of adsorption of the tested complex of OCIF and dextran sulfate to the heparin cross-linked column was then calculated according to the following formula:

$$\frac{(c)}{(a) + (b) + (c)}$$

The results are summarized in Table 5 below for 7 of the complexes prepared in Example 1 above. The corresponding result for non-complexed OCIF is also given. As can be seen from the table, non-complexed OCIF bound more strongly to the heparin column than the complexes of the present invention. It was also found that the complexes of the present invention can be further characterized by their degree of adsorption to a heparin cross-linked column.

Table 5

| Preparation | The degree of adsorption of the OCIF/DS complex to a heparin cross-linked column |
|---|---|
| Prep. 6 | 0.451 |
| Prep. 7 | 0.183 |
| Prep. 8 | 0.153 |
| Prep. 22 | 0.264 |
| Prep. 24 | 0.072 |
| Prep. 25 | 0.611 |
| Prep. 27 | 0.141 |
| OCIF | 0.998 |

Example 7

Immunological Detection of an OCIF/Dextran Sulfate Complex

7(a) Measurement of the Amount of Protein

The amount of protein contained in a complex preparation of Example 1 above was determined according to the method described in Example 5(c) above.

7(b) Immunological Measurement of the Amount of OCIF

The amount of OCIF contained in a complex preparation of Example 1 above determined by immunological means was determined by the ELISA technique described in Example 6 above.

7(c) Calculation of the Immunological Detection Rate

The value obtained in Example 7(b) above was divided by the corresponding value obtained in Example 7(a) above, and the resulting value thus obtained was referred to as "the immunological detection rate".
The results are summarized in Table 6 below. The corresponding result for non-complexed OCIF is also given. It was also found that the complexes of the present invention can be further characterized by their immunological detection rate.

Table 6

| Preparation | The immunological detection rate of OCIF/DS complex. |
|---|---|
| Prep. 6 | 1.07 |
| Prep. 7 | 0.74 |
| Prep. 8 | 0.88 |
| Prep. 22 | 1.06 |
| Prep. 24 | 1.02 |
| Prep. 25 | 0.87 |
| Prep. 27 | 1.04 |
| OCIF | 1.06 |

Reference Example 1

Preparation of a Combination of OCIF and Dextran Sulfate

A combination of OCIF and dextran sulfate sodium salt (molecular weight 5000 or 10000) was prepared as follows using the procedure disclosed in Example 1 of EP-A-1127578 (WO-A-2000/24416).
Purified dimeric human OCIF having a molecular weight of about 120000, prepared as described in Example 1(a) above, was dissolved in 10 mM sodium phosphate buffer solution (pH 6.0) containing 0.15 M sodium chloride and 0.01 % of polysorbate 80 to give a solution having an OCIF concentration of 0.25 mg/ml. DS 5000 (manufactured by Wako Pure Chemical Industries, Ltd.), described in Example 2 above or dextran sulfate sodium salt having a molecular weight of 10000 (manufactured by Wako Pure Chemical Industries, Ltd., hereinafter referred to as "DS10000") was dissolved in the resulting aqueous solution to give a solution having a final concentration of the dextran sulfate sodium salt of 1 or 4 mg/ml, and then sodium hydroxide was added thereto to give a final pH of 7. The aqueous solutions thus obtained were incubated at 4 °C for 24 hours to give the desired preparations containing OCIF and DS5000 or DS10000, which were then used for comparison purposes in Test Example 1 below.
The preparation conditions for each combination are summarized in Table 7 below.

Table 7

| Ref. Prep. Number | Dextran sulfate | | OCIF Conc. (mg/ml) | Temp. (°C) | pH | Incubation time (hours) |
|---|---|---|---|---|---|---|
| | type | Conc. (mg/ml) | | | | |
| Ref.Prep.1 | DS5000 | 4 | 0.25 | 4 | 7 | 24 |
| Ref.Prep.2 | DS10000 | 1 | 0.25 | 4 | 7 | 24 |

Test Example 1

Measurement of the Serum Concentration of Complexes Comprising OCIF and Dextran Sulfate

1(a) Injection and Blood Collection

Five-week old Wistar female rat (having a body weight of about 100 g) were made to abstain from food overnight. The preparation of OCIF and dextran sulfate prepared in either example 1, example 2 or reference example 1 which was to be tested was diluted to a concentration of 0.25 mg/ml with PBS (pH 7.4) containing 0.01 % Polysorbate 80 to prepare an injectable solution, which was then administered to the tail of one of the test rats via a vein in a single dose at an injected level of 2 ml/kg body weight. 6 hours after administration, blood was taken from the heart of the rat.

1(b) Fractionation of Serum

After allowing the blood collected in 1(a) above to coagulate at room temperature for 30 minutes, serum was obtained therefrom as a supernatant by centrifugation of the blood at 14000 rpm for 3 minutes using a rotor with a diameter of 10 cm.

1(c) Quantification of OCIF in the Serum

100 µl of a solution wherein anti-human OCIF monoclonal antibody OI-19 (see EP-A-0974671/WO-A-99/15691) were dissolved in 0.1 M sodium hydrogen carbonate solution to a final OCIF concentration of 10 µg/ml were added to each well of a 96-well micro titre plate (Maxisorp: manufactured by NUNC), and then the plate was sealed and allowed to stand overnight at 4 °C. The antibody solution was then removed by decantation, and 300 µl of a blocking buffer solution (50 % Block Ace: purchased from Dainippon Pharmaceutical Co., Ltd.) were added to each well and then the plate was allowed to stand at room temperature for 2 hours. At the end of this time, each well was washed three times with 300 µl of PBS (pH 7.4) containing 0.1 % Polysorbate 20.
100 µl of purified water and 120 µl of a dilution buffer solution [composition: 0.2 M Tris-hydrochloric acid, 40 % Block Ace (purchased from Dainippon Pharmaceutical Co., Ltd.), 10 µg/ml mouse immunoglobulin G, and 0.1 % polysorbate 20: pH 7.4] were added to 20 µl of the serum to be tested that was collected as described in 1(b) above, and mixed. As a control, 100 µl of purified water and 120 µl of dilution buffer containing human OCIF dimer at a known concentration were added to 20 µl of distilled water, and mixed.
100 µl of each of the serum preparations thus obtained were added to each well, and the plate was then allowed to stand at room temperature for 2 hours. Each well was washed six times after the reaction was complete with 300 µl of a solution containing 0.1% Polysorbate 20 (pH 7.4). 100 µl of a solution obtained by diluting 1000-fold the POD-OI-4 stock solution obtained in Example 4(a) above with a dilution solution [comprising 0.1 M Tris-hydrochloric acid, 25% Block Ace (purchased from Dainippon Pharmaceutical Co., Ltd.), 10 µg/ml mouse immunoglobulin G and 0.1 % Polysorbate 20 (pH 7.4)] were then added to each well, and the plate was allowed to stand at room temperature for 2 hours.
At the end of this time, each well was washed six times with 300 µl of PBS (pH 7.4) containing 0.1 % Polysorbate 20. 100 µl of a substrate solution (TMB soluble reagent: manufactured by Scytek) were then added to each well, and the plate was allowed to stand at room temperature for 10 to 15 minutes. 100 µl of a reaction stop solution (TMB stop buffer: manufactured by Scytek) were then added to each well.
After stirring gently using a shaking machine (Micro plate mixer NS-P: manufactured by Iuchi Seiei-Do Co Ltd.), the absorbance of each well at a wavelength of 450 nm was measured using a micro plate reader (SPECTRA THERMO: manufactured by TECAN). The OCIF concentration in the tested serum was then calculated from a calibration curve created using the standard OCIF solution. The dose was calculated as the dose of OCIF per kg body weight (mg/kg) by measuring the concentration of OCIF in each injection prepared in 1(a) in a similar manner to the case of the serum.

1(d) Serum Concentration

The OCIF in the serum obtained in 1(b) above was quantified for each sample according to the method described in 1(c) above. The results are shown in the Table 8 below.

Table 8

| Preparation | Dose (OCIF mg/kg) | Serum concentration (OCIF ng/ml) | Corrected serum concentration* (OCIF ng/ml) |
|---|---|---|---|
| Prep. 1 | 0.5 | 213 | |
| Prep. 2 | 0.5 | 350 | |
| Prep. 3 | 0.5 | 191 | |
| Prep. 4 | 0.5 | 370 | |
| Prep. 5 | 0.5 | 305 | |
| Prep. 6 | 0.5 | 209 | |
| Prep. 7 | 0.5 | 371 | |
| Prep. 8 | 0.5 | 571 | |
| Prep. 9 | 0.5 | 164 | |
| Prep.10 | 0.5 | 174 | |
| Prep 11 | 0.5 | 235 | |
| Prep. 12 | 0.5 | 249 | |
| Prep. 13 | 0.5 | 177 | |
| Prep. 14 | 0.5 | 271 | |
| Prep. 15 | 0.5 | 313 | |
| Prep. 16 | 0.5 | 359 | |
| Prep. 17 | 0.5 | 269 | |
| Prep. 18 | 0.6 | 400 | 351 |
| Prep. 19 | 0.4 | 526 | 614 |
| Prep. 20 | 0.4 | 553 | 760 |
| Prep. 21 | 0.1 | 132 | 611 |
| Prep. 22 | 0.6 | 752 | 651 |
| Prep. 23 | 0.5 | 340 | |
| Prep. 24 | 0.5 | 830 | |
| Prep.25 | 0.5 | 165 | |
| Prep. 26 | 0.5 | 574 | |
| Prep. 27 | 0.5 | 584 | |
| Prep.28 | 0.5 | 228 | |
| Prep. 29 | 0.5 | 231 | |
| Prep. 30 | 0.5 | 620 | |
| Prep. 31 | 0.5 | 338 | |
| Prep. 32 | 0.5 | 774 | |

* Corrected concentration in serum is the OCIF concentration in serum when converting the dose of OCIF per kg body weight to 0.5 mg/kg.

Table 8   (continued)

| Preparation | Dose (OCIF mg/kg) | Serum concentration (OCIF ng/ml) | Corrected serum concentration* (OCIF ng/ml) |
|---|---|---|---|
| Prep. 33 | 0.5 | 879 | |
| Prep. 34 | 0.5 | 667 | |
| Prep. 35 | 0.2 | 318 | 795 |
| Prep. 36 | 0.1 | 114 | 570 |
| Prep. 37 | 0.5 | 535 | |
| Prep. 38 | 0.4 | 631 | 789 |
| Prep. 39 | 0.5 | 366 | |
| Prep. 40 | 0.5 | 423 | |
| Prep. 41 | 0.4 | 423 | 508 |
| Ref.Prep. 1 | 0.5 | 75 | |
| Ref.Prep. 2 | 0.5 | 24 | |

* Corrected concentration in serum is the OCIF concentration in serum when converting the dose of OCIF per kg body weight to 0.5 mg/kg.

As shown in Table 8, the serum concentrations of the preparations of the present invention administered at a dose of 0.5 mg/kg body weight six hours after administration were 2.2 to 11.7 times higher than that obtained after administration of Reference Preparation 1 with the same dose.

As demonstrated above, complexes of the present invention comprising at least one OCIF, an analogue or a variant thereof and at least one polysaccharide or a variant therof are retained in the blood after administration at a significantly higher concentration when compared with know combinations containing OCIF and polysaccharides, such as those disclosed in WO-A-2000/24416. The complexes of the present invention are useful for preventing or treating various bone metabolic diseases such as osteoporosis, hypercalcemia, bone lytic metastasis, bone loss due to rheumatoid arthritis, osteopenia due to steroid medication, multiple myeloma, osteopenia or hypercalcemia due to renal dysfunction, renal osteodystrophy, osteoarthritis and the like.

## Claims

1. A complex comprising at least one substance selected from the group consisting of osteoclastogenesis inhibitory factor (OCIF), analogues thereof and variants thereof, which is bound to at least one substance selected from the group consisting of polysaccharides and derivatives thereof.

2. A complex according to claim 1, wherein said substance selected from the group consisting of OCIF, analogues thereof and variants thereof is natural type or recombinant type.

3. A complex according to claim 1 or claim 2, wherein said substance selected from the group consisting of OCIF, analogues thereof and variants thereof is a monomer or a dimer.

4. A complex according to claim 3, wherein said substance selected from the group consisting of OCIF, analogues thereof and variants thereof is human monomeric OCIF having a molecular weight as measured by SDS-PAGE under non-reducing conditions of about 60000 or human dimeric OCIF having a molecular weight of about 120000 as measured by SDS-PAGE under non-reducing conditions.

5. A complex according to any one of claims 1 to 4, wherein said OCIF comprises amino acids -21 to +380 of SEQ. ID. NO.1 of the sequence listing.

6. A complex according to any one of claims 1 to 4, wherein said OCIF comprises amino acids +1 to +380 of SEQ. ID. NO.1 of the sequence listing.

7. A complex according to any one of claims 1 to 6, wherein said polysaccharides and derivatives thereof are selected from the group consisting of hyaluronic acid, chondroitin sulfuric acid, dermatan acid, heparan acid, keratan acid, carrageenan, pectin, heparin, dextran and derivatives thereof.

8. A complex according to claim 7, wherein said polysaccharide derivative is selected from dextran sulfate and salts thereof.

9. A complex according to claim 8, wherein said polysaccharide derivative is a sodium salt of dextran sulfate.

10. A complex according to claim 9, wherein the average molecular weight of said dextran sulfate is from 1500 to 12000.

11. A complex according to claim 9, wherein the average molecular weight of said dextran sulfate is from 1800 to 6000.

12. A complex according to any one of claims 1 to 11, wherein the molecular ratio of said substance selected from the group consisting of OCIF, analogues thereof and variants thereof to said substance selected from the group consisting of polysaccharides and derivatives thereof is from 1:1 to 1:10.

13. A complex according to claim 12, wherein said molecular ratio is from 1:1 to 1:8.

14. A complex according to any one of claims 1 to 13, wherein the strength of adsorption of said complex comprising OCIF or an anlogue or variant thereof to heparin is lower than the strength of adsorption of the free, non-complexed OCIF or analogue or variant thereof.

15. A complex according to claim 14, wherein the degree of adsorption to heparin, calculated according to the following procedure, is less than 0.7:

   (a) a column packed with cross-linked agarose beads on which has been immobilized heparin is equilibrated with a low ionic strength buffer containing 0.1 to 0.8 M sodium chloride;
   (b) the complex that is being tested is dissolved in the same low ionic strength buffer as used in (a) and applied to the column and a first eluate is then collected (fraction A);
   (c) the column is then washed further with the same low ionic strength buffer as used in step (a) and a second eluate is collected (fraction B).
   (d) the column is then washed with a buffer having a high ionic strength containing 1.0 to 2.0 M sodium chloride and a third eluate is then collected (fraction C);
   (e) the amount of the complex present in each of fractions A, B and C [designated (a), (b) and (c) respectively] is determined by an immunoassay; and
   (f) the degree of adsorption of the complex to heparin is then determined according to the following formula:

$$\text{degree of adsorption} = \frac{(c)}{(a) + (b) + (c)}$$

16. A complex according to any one of claims 1 to 15 comprising an OCIF or an analogue or variant thereof and dextran sulfate or a salt thereof, wherein the ratio of the number of molecules of said OCIF or analogue or variant thereof present in said complex as determined by enzyme-linked immunosorbent assay (ELISA) using anti-human OCIF monolclonal antibody OI-19 purified from a culture of a hybridoma producing antibody OI-19 (FERM BP-6420) as the antibody bound to the solid phase and anti-human OCIF monoclonal antibody OI-4 purified from a culture of a hybridoma producing antibody OI-4 (FERM BP-6419) labeled with peroxidase in the mobile phase to the number of molecules of OCIF or analogue or variant thereof present in said complex as determined by measuring the total protein content using Lowry's method is from 0.5 to 1.2.

17. A complex according to claim 16, wherein said ratio is from 0.6 to 1.1.

18. A complex according to claim 16, wherein said ratio is from 0.7 to 1.1.

19. A complex according to claim 1, wherein said substance selected from the group consisting of OCIF, analogues thereof and variants thereof is human monomeric OCIF having a molecular weight as measured by SDS-PAGE under non-reducing conditions of about 60000 or human dimeric OCIF having a molecular weight of about 120000 as measured by SDS-PAGE under non-reducing conditions and said polysaccharides and derivatives thereof are selected from the group consisting of hyaluronic acid, chondroitin sulfuric acid, dermatan acid, heparan acid, keratan acid, carrageenan, pectin, heparin, dextran and derivatives thereof, the molecular ratio of said substance selected from the group consisting of OCIF, analogues thereof and variants thereof to said substance selected from the group consisting of polysaccharides and derivatives thereof being from 1:1 to 1:10.

**20.** A complex according to claim 1, wherein said substance selected from the group consisting of OCIF, analogues thereof and variants thereof is human monomeric OCIF having a molecular weight as measured by SDS-PAGE under non-reducing conditions of about 60000 or human dimeric OCIF having a molecular weight of about 120000 as measured by SDS-PAGE under non-reducing conditions and said polysaccharides and derivatives thereof are selected from the group consisting of dextran sulfate and salts thereof, the molecular ratio of said substance selected from the group consisting of OCIF, analogues thereof and variants thereof to said substance selected from the group consisting of polysaccharides and derivatives thereof being from 1:1 to 1:10.

**21.** A complex according to claim 1, wherein said substance selected from the group consisting of OCIF, analogues thereof and variants thereof is human monomeric or dimeric OCIF in which said monomer or one of the units of said OCIF dimer comprises amino acids +1 to +380 of SEQ. ID. NO.1 of the sequence listing and said polysaccharide derivative is a sodium salt of dextran sulfate having an average molecular weight of from 1500 to 12000, the molecular ratio of said substance selected from the group consisting of OCIF, analogues thereof and variants thereof to said sodium salt of dextran sulfate being from 1:1 to 1:10.

**22.** A complex according to claim 21, wherein the molecular ratio of said substance selected from the group consisting of OCIF, analogues thereof and variants thereof to said sodium salt of dextran sulfate being from 1:1 to 1:8.

**23.** A complex according to claim 21, wherein the molecular ratio of said substance selected from the group consisting of OCIF, analogues thereof and variants thereof to said sodium salt of dextran sulfate being from 1:1 to 1:5.

**24.** A complex according to any one of claims 21 to 23, wherein said polysaccharide derivative is a sodium salt of dextran sulfate having an average molecular weight of from 1800 to 6000.

**25.** A method for prolonging the time that OCIF or an analogue or variant thereof is retained in the bloodstream after administration to a patient by complexing prior to administration at least one of said OCIF, said analogue thereof or said variant thereof as defined in any one of claims 1 to 6 with at least one polysaccharide or a derivative thereof as defined in any one of claims 1 and 7 to 11.

**26.** A pharmaceutical composition comprising an effective amount of a pharmacologiocally active agent together with a carrier or diluent therefor, wherein said pharmacologiocally active agent is a complex according to any one of claims 1 to 24.

**27.** A pharmaceutical composition according to claim 26 for the treatment or prophylaxis of bone metabolic diseases.

**28.** Use of a complex according to any one of claims 1 to 24 in the manufacture of a medicament for the prophylaxis or treatment of bone metabolic diseases.

**29.** Use according to claim 28, wherein said bone metabolic diseases are selected from the group consisting of osteoporosis, osteopenia, Paget's disease, osteomyelitis, infectious focus due to loss of bone, hypercalcemia, osteoclasis, joint destruction or osteopenia due to rheumatism, osteoarthritis, loss of periodontal bone, cancer metastasis of bone, osteonecrosis or osteocyte death accompanying traumatic injury, Gaucher's disease, sickle cell anemia, lupus erythematosus systemic or nontraumatic injury, osteodystrophy, and cachexia due to solid carcinoma or cancer metastasis of bone or hemology-malignant disease.

**30.** A method for the preparation of a complex according to any one of claims 1 to 24, said method comprising the steps of incubating at least one substance selected from the group consisting of OCIF, analogues thereof and variants thereof as defined in any one of claims 1 to 6 with at least one substance selected from the group consisting of polysaccharides and derivatives thereof as defined in any one of claims 1 and 7 to 11 at a pH of from 9.5 to 12 and then removing any free polysaccharides or derivatives thereof that are not bound to said OCIF, analogues thereof or variants thereof.

**31.** A method according to claim 30, wherein the incubation of said at least one substance selected from the group consisting of OCIF, analogues thereof and variants thereof with said at least one substance selected from the group consisting of polysaccharides and derivatives thereof is performed at a pH of from 10 to 11.

**32.** A method according to claim 30 or claim 31, wherein any free polysaccharides or derivatives thereof that are not bound to said OCIF, analogues thereof or variants thereof after incubation are removed by gel filtration chromatography.

**33.** A complex as defined in any one of claims 1 to 24, said complex being prepared by a method according to any one of claims 30 to 32.